# EUROPEAN PATENT SPECIFICATION

(11) **EP 2 767 595 B1**
(45) Date of publication and mention of the grant of the patent: **19.09.2018**
(21) Application number: 13161597.3
(22) Date of filing: 22.05.2007
(51) Int. Cl.: C12Q 1/68, G01N 33/48, G01N 33/574, G06F 19/00

(54) **Detection method for characterising the anatomical origin of a cell**
Nachweisverfahren zur Bestimmung des anatomischen Ursprungs einer Zelle
Procédé de détection permettant la détermination de l'origine anatomique d' une cellule

(30) Priority: 22.05.2006 US 802312 P
(43) Date of publication of application: 20.08.2014
(62) Divisional of application: 07718949.6
(73) Proprietor: Clinical Genomics Pty Ltd, North Ryde, New South Wales 2113 (AU); Commonwealth Scientific and Industrial Research Organisation, Campbell, Australian Capital Territory 2612 (AU)
(72) Inventor: Lapointe, Lawrence, Kings Langley, New South Wales 2147 (AU); Dunne, Robert, Darlington, New South Wales 2008 (AU)
(74) Representative: Elsy, David

(56) References cited:
- GLEBOV OLEG K ET AL: "Distinguishing Right form left Colon by the Pattern of Gene Expression", CANCER EPIDEMIOLOGY, BIOMARKERS AND PREVENTION, PHILADELPHIA, PA, US, vol. 12, no. 8, 1 August 2003 (2003-08-01) , pages 755-762, XP008090436, ISSN: 1055-9965
- KOMURO K ET AL: "Right- and left-sided colorectal cancers display distinct expression profiles and the anatomical stratification allows a high accuracy prediction of lymph node metastasis", JOURNAL OF SURGICAL RESEARCH, ACADEMIC PRESS INC., SAN DIEGO, CA, US LNKD- DOI:10.1016/J.JSS.2004.10.009, vol. 124, no. 2, 1 April 2005 (2005-04-01) , pages 216-224, XP004835905, ISSN: 0022-4804

## Description

### FIELD OF THE INVENTION

The present invention relates generally to an array of nucleic acid molecules, the expression profiles of which characterise the anatomical origin of a cell or population of cells within the large intestine. More particularly, the present invention relates to an array of nucleic acid molecules, the expression profiles of which characterise the proximal or distal origin of a cell or population of cells within the large intestine. The expression profiles of the present invention are useful in a range of applications including, but not limited to determining the anatomical origin of a cell or population of cells which have been derived from the large intestine. Still further, since the progression of a normal cell towards a neoplastic state is often characterised by phenotypic de-differentiation, the method of the present invention also provides a means of identifying a cellular abnormality based on the expression of an incorrect expression profile relative to that which should be expressed by the subject cells when considered in light of-their anatomical location within the colon. Accordingly, this aspect of the invention provides a valuable means of identifying the existence of large intestine colon cells, these being indicative of an abnormality within the large intestine such as the onset or predisposition to the onset of a condition such as a colorectal neoplasm.

### BACKGROUND OF THE INVENTION

Bibliographic details of the publications referred to by author in this specification are collected alphabetically at the end of the description.

The reference to any prior art in this specification is not, and should not be taken as, an acknowledgment or any form of suggestion that that prior art forms part of the common general knowledge in Australia.

Adenomas are benign tumours of epithelial origin which are derived from glandular tissue or exhibit clearly defined glandular structures. Some adenomas show recognisable tissue elements, such as fibrous tissue (fibroadenomas), while others, such as bronchial adenomas, produce active compounds giving rise to clinical syndromes. Tumours in certain organs, including the pituitary gland, are often classified by their histological staining affinities, for example eosinophil, basophil and chromophobe adenomas.

Adenomas may become carcinogenic and are then termed adenocarcinomas. Accordingly, adenocarcinomas are defined as malignant epithelial tumours arising from glandular structures, which are constituent parts of most organs of the body. This term is also applied to tumours showing a glandular growth pattern. These tumours may be subclassified according to the substances that they produce, for example mucus secreting and serous adenocarcinomas, or to the microscopic arrangement of their cells into patterns, for example papillary and follicular adenocarcinomas. These carcinomas may be solid or cystic (cystadenocarcinomas). Each organ may produce tumours showing a variety of histological types, for example the ovary may produce both muconous and cystadenocarcinoma. In general, the overall incidence of carcinoma within an adenoma is approximately 5%. However, this is related to size and although it is rare in adenomas of less than 1 centimetre, it is estimated at 40 to 50% among villous lesions which are greater than 4 centimetres. Adenomas with higher degrees of dysplasia have a higher incidence of carcinoma. Once a sporadic adenoma has developed, the chance of a new adenoma occurring is approximately 30% within 26 months.

Colorectal adenomas represent a class of adenomas which are exhibiting an increasing incidence, particularly in more affluent countries. The causes of adenoma, and its shift to adenocarcinoma, are still the subject of intensive research. To date it has been speculated that in addition to genetic predisposition, environmental factors (such as diet) play a role in the development of this condition. Most studies indicate that the relevant environmental factors relate to high dietary fat, low fibre and high refined carbohydrates.

Colonic adenomas are localised proliferations of dysplastic epithelium which are initially flat. They are classified by their gross appearance as either sessile (flat) or penduculated (having a stalk). While small adenomas (less than 0.5 millimetres) exhibit a smooth tan surface, penduculated adenomas have a head with a cobblestone or lobulated red-brown surface. Sessile adenomas exhibit a more delicate villous surface. Penduculated adenomas are more likely to be tubular or tubulovillous while sessile lesions are more likely to be villous. Sessile adenomas are most common in the cecum and rectum while overall penduculated adenomas are equally split between the sigmoid-rectum and the remainder of the large intestine.

Adenomas are generally asymptomatic, therefore rendering difficult their early diagnosis and treatment. It is technically impossible to predict the presence or absence of carcinoma based on the gross appearance of adenomas, although larger adenomas are thought to exhibit a higher incidence of concurrent malignancy than smaller adenomas. Sessile adenomas exhibit a higher incidence of malignancy than penduculated adenomas of the same size. Some adenomas result in the production of microscopic stool blood loss. However, since stool blood can also be indicative of non-adenomatous conditions and obstructive symptoms are generally not observed in the absence of malignant change, the accurate diagnosis of adenoma is rendered difficult without the application of highly invasive procedures such as biopsy analysis. Accordingly, there is an on-going need to elucidate not only the causes of adenoma and its shift to malignancy but to develop more informative diagnostic protocols, in particular protocols which will enable the rapid, routine and accurate diagnosis of adenoma and adenocarcinoma at an early stage, such as the pre-malignant stage. To this end, studies of colorectal adenocarcinoma have suggested a variable incidence, histopathology and prognosis between proximal and distal tumours.

In terms of pursuing this line of investigation, the advent of gene expression profiling has led to an improved understanding of intestinal mucosa development. For example, regulation of transcription factors involved in producing and maintaining the radial-axis balance from the crypt base to the lumen and those giving rise to epithelial cell differentiation are now better understood as a result of microarray gene expression analysis. [Peifer, 2002, Nature 420: 274-5,277; Traber, 1999, Adv Exp Med Biol 470:1-14]. Similarly, understanding has improved of the developmentally programmed genetic events within the embryonic gut, especially those molecular control mechanisms responsible for regional epithelium differences between the small intestine and large intestine. [de Santa Barbara et al., 2003, Cell Mol Life Sci 60:1322-1332; Park et al., 2005, Genesis 41:1-12] On the other hand, little is known about the proximal-distal gene expression variation along the longitudinal axis of the large intestine. [Bates et al. 2002, Gastroenterology 122:1467-1482] Epidemiologic studies of colorectal adenocarcinoma suggest support for variable incidence, histopathology, and prognosis between proximal and distal tumours. [Bonitlion-Kopp and Benhamiche, 1999, Eur J Cancer Prev 8 Suppl 1 :S3-12; Bufill, 1990, Ann Intern Med 113:779-788; Deng et al., 2002, Br J Cancer 86:574-579; Distler and Holt, 1997, Dig Dis 15:302-311]. Thus an understanding of location-specific variation could provide valuable insight into those diseases that have characteristic distribution patterns along the colorectum, including colorectal cancer. [Birkenkamp-Demtroder et al., 2005, Gut 54:374-384; Caldero et al., 1989, Virchows Arch A Pathol Anat Histopathol 415:347-356; Garcia-Hirschfeld Garcia et al., 1999, Rev Esp Enferm Dig 91:481-488].

The colorectum (also termed the large intestine) is often divided for clinical convenience into six anatomical regions starting from the terminal region of the ileum: the cecum; the ascending colon; the transverse colon; the descending colon, the sigmoid colon; and the rectum. Alternatively, these segments may be grouped to divide the large intestine into a two region model comprising the proximal and distal large intestine. The proximal ("right") region is generally taken to include the cecum, ascending colon, and the transverse colon while the distal ("left") region includes the splenic flexure, the descending colon, the sigmoid flexure and the rectum. This division is supported by the distinct embryonic ontogenesis of these regions whose junction is two thirds along the transverse colon and also by the distinct arterial supply to each region. While the proximal large intestine develops from the embryonic midgut and is supplied by the superior mesenteric artery, the distal large intestine forms from the embryonic hindgut and is supplied by the inferior mesenteric artery. [Yamada and Alpers, 2003, Textbook of Gastroenterology, 2 Vol. Set.] A comprehensive of review of proximal distal differences are provided in [Iacopetta, 2002, Int J Cancer 101:403-408].

In work leading up to the present invention it has been determined that a panel of genes are differentially expressed between the proximal and distal sections of the human large intestine. Accordingly, this has enabled the development of means for determining whether a large intestine derived cell of interest is of proximal origin or distal origin. Samples of normal large intestine derived cells or tissues can therefore be routinely characterised in terms of their anatomical origin within the large intestine. Still further, since most disease conditions are characterised by some change in phenotypic profile or gene transcription of the diseased cells, this being particularly true of cells which are predisposed to or have become neoplastic, the method the present invention provides a convenient means of identifying abnormal cells or cells which are predisposed to becoming abnormal. More particularly, where a cell of known large intestine anatomical origin expresses one or more genes or profiles of genes which are not characteristic of that location, the cell is classified as abnormal and may then undergo further analysis to elucidate the nature of that abnormality.

### SUMMARY OF THE INVENTION

Hsoagtout this specification and the claims which follow, unless the context requires otherwise, the word "comprise", and variations such as "comprises" and "comprising", will be understood to imply the inclusion of a staled integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

One aspect of the present invention is directed to a method for determining the anatomical origin of a cell or cellular population derived from either the proximal or distal region of the large intestine of an individual, said method comprising measuring the level of expression of one or more gene or genes selected from:
(i) the gene or genes detected by Affymetrix probe number 225457_s_at; and
(ii) the gene or genes detected by Affymetrix probe number 225458_at;
in said cell or cellular population wherein a higher level of expression of the genes relative to normal distal large intestine control levels is indicative of a proximal large intestine origin, and wherein said proximal region comprises the cecum and the ascending colon and said distal region comprises the splenic flexure, descending colon, sigmoid flexure and rectum.

In another aspect there is provided a method of determining the onset or predisposition to the onset of a cellular abnormality or a condition characterised by a cellular abnormality in the large intestine, said method comprising determining, in accordance with the methods hereinbefore described, the proximal-distal gene expression profile of a biological sample derived from a known proximal or distal origin in the large intestine wherein the detection of a gene expression profile which is inconsistent with the normal proximal-distal large intestine gene expression profile is indicative of the abnormality of the cell or cellular population expression said profile.

### BRIEF DESCRIPTION OF THE DRAWINGS

**Figure 1** is a graphical representation of the comparison of the number of differential probesets when the divide between proximal and distal regions is moved.
**Figure 2** is a graphical representation of the relative number of transcripts elevated in proximal and distal large intestine.
**Figure 3** is a graphical representation of a typical example of a two-gene model.
**Figure 4** is a graphical representation of the relative direction of increasing expression of transcripts that exhibit a gradual change along the colorectum.
**Figure 5** is a graphical representation of genes exhibiting five-segment model behaviour.
**Figure 6a** is a graphical representation of a typical example of the first and second principal components generated by applying principal component analysis (PCA) to all 44,928 probesets of the Discover data set, revealing little, if any, structure;
**Figure 6b** is a graph of the first and second principal components generated by applying PCA to a subset of 115 probesets that are each differentially expressed in tissue samples from the cecum and rectum (i.e., the extreme proximal and distal ends of the large intestine), revealing two classes corresponding to the proximal and distal portions of the large intestine;
**Figure 7A** is a graph of the first principal component of Figure 6A as a function of tissue location along the proximal-distal axis of the large intestine;
**Figure 7B** is a graph of the first principal component of Figure 6B as a function of tissue location along the proximal-distal axis of the large intestine;
**Figure 8A** is a graph of the first and second canonical variates generated by profile analysis;
**Figure 8B** is a graph of the first canonical variate of Figure 8A as a function of tissue location along the proximal-distal axis of the large intestine;
**Figure 9** is a graph of the cross-validated error estimates of support vectors generated from respective subsets of genes as a function of the number of genes in each subset;
**Figure 10** is a block diagram of a preferred embodiment of a detection system; and
**Figure 11** is a flow diagram of a preferred embodiment of a detection method executed by the detection system.

### DETAILED DESCRIPTION OF THE INVENTION

The present invention is predicated, in part, on the elucidation of gene expression profiles which characterise the anatomical origin of a cell or cellular population from the large intestine in terms of a proximal origin versus a distal origin. This finding has now facilitated the development of routine means of characterising, in terms of its anatomical origin, a cellular population derived from the large intestine. Still further, since some cellular disorders are characterised by a change in the gene expression profile of the diseased cell relative to a corresponding normal cell, the present invention also provides a means of routinely screening large intestine cells, which have been derived from a known anatomical location within the large intestine, for any changes to the gene expression profile which they would be expected to express based on that particular location. Where the correct gene expression profile is not observed, the cell is exhibiting an abnormality and should be further assessed by way of diagnosing the specifics of the abnormality. In particular, it would be appreciated by the person of skill in the art that neoplastic cells, or cells predisposed thereto, sometimes undergo de-differentiation - this being evidenced by a change to the gene expression phenotype of the cell to a less differentiated phenotype. Accordingly, any change to the gene expression profile characteristic of a large intestine cell of proximal or distal origin may be indicative of the onset or predisposition to the onset of a large intestine neoplasma, such as an adenoma or an adenocarcinoma. Also described are nucleic acid arrays, such as microarrays, for use in the method of the invention.

Accordingly, one aspect of the present invention if directed to a method as defined in claim 1.

As detailed hereinbefore, the method of the present invention is predicated on the determination that distal versus proximal location of a cell within the large intestine can now be ascertained by virtue of gene expression profiles which are unique to the cells of each of these locations. Accordingly, reference to determining the "anatomical origin" or anatomical location" of a cell or cellular population "derived from the large intestine" should be understood as a reference to determining whether the cell in issue originates from the distal region of the large intestine or the proximal regional of the larger intestine. Further to this, by "origin" or "location" is meant the location of the cell or cells under investigation either just prior to the time that cell was harvested from the large intestine or, where the cell has naturally detached from the large intestine (e.g. whether it has sloughed off and is found in a stool sample), at the time immediately prior to the cell detaching from the large intestine. Without limiting the present invention to any one theory or mode of action, the large intestine has no digestive function, as such, but absorbs large amounts of water and electrolytes from the undigested food passed on from the small intestine. At regular intervals, peristaltic movements move the dehydrated contents (faeces) towards the rectum. For clinical convenience the large intestine is generally divided into six anatomical regions commencing after the terminal region of the ileum-these being:
(i) the cecum;
(ii) the ascending colon;
(iii) the transverse colon;
(iv) the descending colon;
(v) the sigmoid colon; and
(vi) the rectum.

These segments can also be grouped to divide the large intestine into a two region model comprising the proximal and distal large intestine. The proximal region is generally understood to include the cecum and ascending colon while the distal region includes the splenic flexure, the descending colon, the sigmoid flexure and the rectum. This division between the proximal and distal region of the large intestine is thought to occur approximately two thirds along the transverse colon. This division is supported by the distinct embryonic ontogenesis of these regions whose junction is two thirds along the transverse colon and also by the distinct arterial supply to each region. Accordingly, tissues of the transverse colon may be either proximal or distal depending on which side of this junction corresponds to their point of origin. It would be appreciated that although the method of the present invention may not necessarily indicate from which part of the proximal or distal large intestine a cell originated, it will provide valuable information in relation to whether the tissue is of proximal origin or distal origin. While the proximal large intestine develops from the embryonic midgut and is supplied by the superior mesenteric artery, the distal large intestine forms from the embryonic hindgut and is supplied by the inferior mesenteric artery.

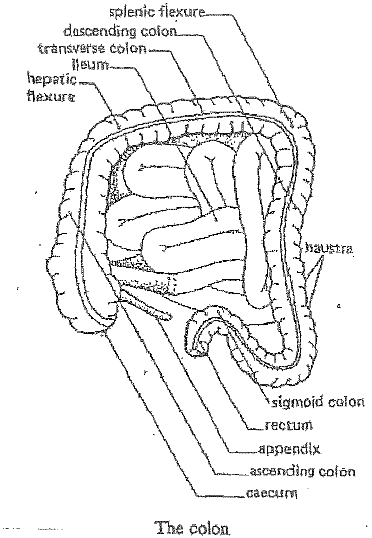
splenic flexure
descending colon
transverse colon
ileum
hepatic
flexure
haustra
sigmoid colon
rectum
appendix
ascending colon
caecum
The colon

Accordingly, reference to the "proximal" region of the large intestine should be understood as a reference to the section of the large intestine comprising the cecum and ascending colon, while reference to the "distal" region of the large intestine should be understood as a reference to the splenic flexure, descending colon, sigmoid flexure and rectum. The transverse colon region comprises both proximal and distal region, the relative proportions of which will depend on where the junction of the proximal and distal tissue occurs. Specifically, the tissue of the transverse colon can be from either the proximal or distal region depending on the relative distance between the hepatic and splenic flexures.

In accordance with the present invention, it has been determined that the genes detailed in paragraphs (i) and (ii), above, are modulated, in terms of differential changes to their levels of expression depending on whether the cell expressing that gene is located in the proximal region of the large intestine or the distal region of the large intestine. For ease of reference, these genes and their mRNA transcripts are depicted in italicised text while their protein expression products are depicted in non-italicised text These genes are collectively referred to as "location markers".

Each of the genes detailed in sub-paragraphs (i) and (ii), above, would be well known to the person of skill in the art, as would their encoded protein expression products. The identification of these genes as markers of colorectal (large intestine) cell location occurred by virtue of differential expression analysis using Affymetrix HG133A or HG133B gene chips. To this end, each gene chip is characterised by approximately 45,000 probe sets which detect the RNA transcribed from approximately 35,000 genes. On average, approximately 11 probe pairs detect overlapping or consecutive regions of the RNA transcript of a single gene. In general, the gene from which the RNA transcripts are identifiable by the Affymetrix probes are well known and characterised genes. However, to the extent that some of the probes detect RNA transcripts which are not yet defined, these genes are indicated as "the gene or genes detected by Affymetrix probe x". In some cases a number of genes may be detectable by a single probe. This is also indicated where appropriate. It should be understood, however, that this is not intended as a limitation as to how the expression level of the subject gene can be detected. In the first instance, it would be understood that the subject gene transcript is also detectable by other probes which would be present on the Affymetrix gene chip. The reference to a single probe is merely included as an identifier of the gene transcript of interest. In terms of actually screening for the transcript, however, one may utilise a probe directed to any region of the transcript and not just to the terminal 600bp transcript region to which the Affymetrix probes are generally directed.

Reference to each of the genes detailed above and their transcribed and translated expression products should therefore be understood as a reference to all forms of these molecules and to fragments, mutants or variants thereof. As would be appreciated by the person of skill in the art, some genes are known to exhibit allelic variation between individuals. Accordingly, the present invention should be understood to extend to such variants which, in terms of the present diagnostic applications, achieve the same outcome despite the fact that minor genetic variants between the actual nucleic acid sequences may exist between individuals. The present invention should therefore be understood to extend to all RNA (eg mRNA, primary RNA transcript, miRNA, tRNA, rRNA etc), cDNA and peptide isoforms which arise from alternative splicing or any other mutation, polymorphic or allelic variation. It should also be understood to include reference to any subunit polypeptides such as precursor forms which may be generated, whether existing as a monomer, multimer, fusion protein or other complex.

Without limiting the present invention to any one theory or mode of action, although each of the genes hereinbefore described is differentially expressed, either singly or in combination, as between the cells of the distal and proximal large intestine, and is therefore diagnostic of the anatomical origin of any given cell sample, the expression of some of these genes exhibited particularly significant levels of sensitivity, specificity, positive predictive value and/or negative predictive value. Accordingly, in a preferred embodiment, one would screen for and assess the expression level of one or more of these genes.

The detection method of the present invention can be performed on any suitable biological sample. To this end, reference to a "biological sample" should be understood as a reference to any sample of biological material derived from an animal such as, but not limited to, cellular material, biofluids (eg. blood), faeces, tissue biopsy specimens, surgical specimens or fluid which has been introduced into the body of an animal and subsequently removed (such as, for example, the solution retrieved from an enema wash). The biological sample which is tested according to the method of the present invention may be tested directly or may require some form of treatment prior to testing. For example, a biopsy or surgical sample may require homogenisation prior to testing or it may require sectioning for *in situ* testing of the qualitative expression levels of individual genes. Alternatively, a cell sample may require permeabilisation prior to testing. Further, to the extent that the biological sample is not in liquid form, (if such form is required for testing) it may require the addition of a reagent, such as a buffer, to mobilise the sample.

To the extent that the location marker gene is present in a biological sample, the biological sample may be directly tested or else all or some of the nucleic acid material present in the biological sample may be isolated prior to testing. In yet another example, the sample may be partially purified or otherwise enriched prior to analysis. For example, to the extent that a biological sample comprises a very diverse cell population, it may be desirable to enrich for a sub-population of particular interest It is within the scope of the present invention for the target cell population or molecules derived therefrom to be pretreated prior to testing, for example, inactivation of live virus or being run on a gel. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation. Preferably, said sample is a faecal sample, enema wash, surgical resection or tissue biopsy.

As detailed hereinbefore, the present invention is designed to characterise a cell or cellular population, which is derived from the large intestine, in terms of its anatomical origin within the large intestine. Accordingly, reference to "cell or cellular population" should be understood as a reference to an individual cell or a group of cells. Said group of cells may be a diffuse population of cells, a cell suspension, an encapsulated population of cells or a population of cells which take the form of tissue.

Reference to "expression" should be understood as a reference to the transcription and/or translation of a nucleic acid molecule. In this regard, the present invention is exemplified with respect to screening for location markers taking the form of RNA transcripts (eg primary RNA, mRNA, miRNA, tRNA, rRNA). Reference to "RNA" should be understood to encompass reference to any form of RNA, such as primary RNA, mRNA, miRNA, tRNA or rRNA. Without limiting the present invention in any way, the modulation of gene transcription leading to increased or decreased RNA synthesis will also correlate with the translation of some of these RNA transcripts (such as mRNA) to produce an expression product. Accordingly, the present invention also extends to detection methodology which is directed to screening for modulated levels or patterns of expression of the location marker expression products as an indicator of the proximal or distal origin of a cell or cellular population. Although one method is to screen for mRNA transcripts and/or the corresponding protein expression product, it should be understood that the present invention is not limited in this regard and extends to screening for any other form of location marker such as, for example, a primary RNA transcript. It is well within the skill of the person of skill in the art to determine the most appropriate screening target for any given situation. Preferably, the protein expression products is the subset of analysis.

Reference to "nucleic acid molecule" should be understood as a reference to both deoxyribonucleic acid molecules and ribonucleic acid molecules. The present invention therefore extends to both directly screening for mRNA levels in a biological sample or screening for the complimentary cDNA which has been reverse-transcribed from an mRNA population of interest. It is well within the skill of the person of skill in the art to design methodology directed to screening for either DNA or RNA. As detailed above, the method of the present invention also extends to screening for the protein expression product translated from the subject mRNA.

The method of the present invention is predicated on the correlation of the expression levels of the location markers of a biological sample with the normal proximal and distal levels of these markers. The "normal level" is the level of marker expressed by a cell or cellular population of proximal origin in the large intestine and the level of marker expressed by a cell or cellular population of distal origin. Accordingly, there are two normal level values which are relevant to the detection method of the present invention. It would be appreciated that these normal level values are calculated based on the expression levels of large intestine derived cells which do not exhibit an abnormality or predisposition to an abnormality which would alter the expression levels or patterns of these markers. The normal level may be determined using tissues derived from the same individual who is the subject of testing. However, it would be appreciated that this may be quite invasive for the individual concerned and it is therefore likely to be more convenient to analyse the test results relative to a standard result which reflects individual or collective results obtained from healthy individuals, other than the patient in issue. This latter form of analysis is in fact the preferred method of analysis since it enables the design of kits which require the collection and analysis of a single biological sample, being a test sample of interest. The standard results which provide the proximal and distal normal reference levels may be calculated by any suitable means which would be well known to the person of skill in the art. For example, a population of normal tissues can be assessed in -terms of the level of expression of the location markers of the present invention, thereby providing a standard value or range of values against which all future test samples are analysed. It should also be understood that the proximal and distal normal reference levels may be determined from the subjects of a specific cohort and for use with respect to test samples derived from that cohort. Accordingly, there may be determined a number of standard values or ranges which correspond to cohorts which differ in respect of characteristics such as age, gender, ethnicity or health status. Said "normal level" may be a discrete level or a range of levels. The results of biological samples which are tested are preferably assessed against both the proximal and distal normal reference levels. An increase in the expression of the genes of group (i), hereinbefore defined, relative to normal distal levels is indicative of the test tissue being of proximal origin while an increase in the expression of the genes of group (ii), hereinbefore defined, relative to normal proximal levels is indicative of the tissue being of distal origin. It would also be appreciated, however, that one may also approach the defined correlative step by analysing the results which are obtained from the point of view of determining whether the result obtained is the same as a normal or distal level, thereby indicating that the test sample is of the same origin as the normal reference level sample against which it has been assessed.

It should be understood that the "individual" who is the subject of testing may be any primate. Preferably the primate is a human.

As detailed hereinbefore, it should be understood that although the present invention is exemplified with respect to the detection of nucleic acid molecules, it also encompasses methods of detection based on testing for the expression product of the subject location markers. The present invention should also be understood to mean methods of detection based on identifying either protein product or nucleic acid material in one or more biological samples. However, it should be understood that some of the location markers may correlate to genes or gene fragments which do not encode a protein expression product. Accordingly, to the extent that this occurs it would not be possible to test for an expression product and the subject marker must be assessed on the basis of nucleic acid expression profiles.

The term "protein" should be understood to encompass peptides, polypeptides and proteins. The protein may be glycosylated or unglycosylated and/or may contain a range of other molecules fused, linked, bound or otherwise associated to-the protein such as amino acids, lipids, carbohydrates or other peptides, polypeptides or proteins. Reference herein to a "protein" includes a protein comprising a sequence of amino acids as well as a protein associated with other molecules such as amino acids, lipids, carbohydrates or other peptides, polypeptides or proteins.

The location marker proteins of the present invention may be in multimeric form meaning that two or more molecules are associated together. Where the same protein molecules are associated together, the complex is a homomultimer. An example of a homomultimer is a homodimer. Where at least one marker protein is associated with at least one non-marker protein, then the complex is a heteromultimer such as a heterodimer.

Reference to a "fragment" should be understood as a reference to a portion of the subject nucleic acid molecule. This is particularly relevant with respect to screening for modulated RNA levels in stool samples since the subject RNA is likely to have been degraded or otherwise fragmented due to the environment of the gut. One may therefore actually by detecting fragments of the subject RNA molecule, which fragments are identified by virtue of the use of a suitability specific probe.

Reference to "proximal-distal origin" should be understood as a reference to cells or expression data of either a proximal origin or a distal origin. Reference to "cells or cellular subpopulations", "large intestine", "proximal", "distal", "origin", "location", "gene" and "expression" should be understood to have the same meaning as hereinbefore provided.

The present disclosure also provides a detection system having components for executing any of the above methods.

The disclosure also provides a computer-readable storage medium having stored thereon program instructions for executing anyone of the above methods.

Accordingly, in another aspect there is provided a method of determining the onset or predisposition to the onset of a cellular abnormality or a condition characterised by a cellular abnormality in the large intestine, said method comprising determining, in accordance with one of the methods hereinbefore described, the proximal-distal gene expression profile of a biological sample derived from a known proximal or distal origin in the large intestine wherein the detection of a gene expression profile which is inconsistent with the normal proximal-distal large intestine gene expression profile is indicative of the abnormality of the cell or cellular population expressing said profile.

Reference to "gene expression profile" should be understood as a reference to the univariate or multivariate gene expression results hereinbefore described. For example, the "profile" may correlate to the expression level of one or more marker genes as hereinbefore discussed or the result of the multivariate analysis of the genes and/or gene sets hereinbefore described. Accordingly, reference to "proximal-distal gene expression profile" is a reference to the gene expression profile characteristic of cells of proximal large intestine origin and that of cells of distal large intestine origin.

It would be appreciated that the cells which are the subject of analysis in the context of the present invention are of known proximal or distal origin. This information may be determined by any suitable method but is most conveniently satisfied by isolating the biological sample from a defined location in the large intestine via a biopsy. However, other suitable methods of harvesting or otherwise determining the anatomical origin of the biological sample are not excluded.

The abnormality of a cell or cellular population of the biological sample is based on the detection of a gene expression profile which is inconsistent with that of the profile which would normally characterise a cell of its particular proximal or distal origin. By "inconsistent" is meant that the expression level of one or more of the genes which are analysed is not consistent with that which is typically observed in a normal control.

The method of the present invention is useful as a one off test or as an on-going monitor of those individuals thought to be at risk of the development of disease or as a monitor of the effectiveness of therapeutic or prophylactic treatment regimes such as the ablation of diseased cells which are characterised by an abnormal gene expression profile. In these situations, mapping the modulation of location marker expression levels or expression profiles in any one or more classes of biological samples is a valuable indicator of the status of an individual or the effectiveness of a therapeutic or prophylactic regime which is currently in use. Accordingly, the method of the present invention should be understood to extend to monitoring for the modulation of location marker levels or expression profiles in an individual relative to a normal level (as hereinbefore defined) or relative to one or more earlier gene marker levels or expression profiles determined from a biological sample of said individual.

Means of testing for the subject expressed location markers in a biological sample can be achieved by any suitable method, which would be well known to the person of skill in the art, such as but not limited to:
(i) *In vivo* detection.
   Molecular Imaging may be used following administration of imaging probes or reagents capable of disclosing altered expression of the markers in the intestinal tissues.
   Molecular imaging (Moore et al., BBA, 1402:239-249, 1988; Weissleder et al., Nature Medicine 6:351-355, 2000) is the *in vivo* imaging of molecular expression that correlates with the macro-features currently visualized using "classical" diagnostic imaging techniques such as X-Ray, computed tomography (CT), MRI, Positron Emission Tomography (PET) or endoscopy.
(ii) Detection of up-regulation of RNA expression in the cells by Fluorescent *In Situ* Hybridization (FISH), or in extracts from the cells by technologies such as Quantitative Reverse Transcriptase Polymerase Chain Reaction (QRTPCR) or Flow cytometric qualification of competitive RT-PCR products (Wedemeyer et al., Clinical Chemistry 48:9 1398-1405, 2002).
(iii) Assessment of expression profiles of RNA from cellular extracts, for example by array technologies (Alon et al., Proc. Natl. Acad. Set USA: 96, 6745-6750, June 1999).
   A "microarray" is a linear or multi-dimensional array of preferably discrete regions, each having a defined area, formed on the surface of a solid support. The density of the discrete regions on a microarray is determined by the total numbers of target polynucleotides to be detected on the surface of a single solid phase support, preferably at least about 50/cm², more preferably at least about 100/cm², even more preferably at least about 500/cm², and still more preferably at least about 1,000/cm². As used herein, a DNA microarray is an array of oligonucleotide probes placed onto a chip or other surfaces used to amplify or clone target polynucleotides. Since the position of each particular group of probes in the array is known, the identities of the target polynucleotides can be determined based on their binding to a particular position in the microarray.
   Recent developments in DNA microarray technology make it possible to conduct a large scale assay of a plurality of target nucleic acid molecules on a single solid phase support. U.S. Pat. No. 5,837,832 (Chee et al.) and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells or tissues, often a tissue of interest and a control tissue, is compared and any differences in gene expression among the respective tissues are identified. Such information is useful for the identification of the types of genes expressed in a particular tissue type and diagnosis of conditions based on the expression profile.
   In one example, RNA from the sample of interest is subjected to reverse transcription to obtain labelled cDNA. See U.S. Pat. No. 6,410,229 (Lockhart et al.) The cDNA is then hybridized to oligonucleotides or cDNAs of known sequence. arrayed on a chip or other surface in a known order. In another example, the RNA is isolated from a biological sample and hybridised to a chip on which are anchored cDNA probes. The location of the oligonucleotide to which the labelled cDNA hybridizes provides sequence information on the cDNA, while the amount of labelled hybridized RNA or cDNA provides an estimate of the relative representation of the RNA or cDNA of interest. See Schena, et al. Science 270:467-470 (1995). For example, use of a cDNA microarray to analyze gene expression patterns in human cancer is described by DeRisi, et al. (Nature Genetics 14:457-460 (1996)).
   In a preferred embodiment, nucleic acid probes corresponding to the subject nucleic acids are made. The nucleic acid probes attached to the biochip are designed to be substantially complementary to the nucleic acids of the biological sample such that specific hybridization of the target sequence and the probes of the present invention occurs. This complementarity need not be perfect, in that there may be any number of base pair mismatches that will interfere with hybridization between the target sequence and the single stranded nucleic acids of the present invention. It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions.
   A nucleic acid probe is generally single stranded but can be partly single and partly double stranded. The strandedness of the probe is dictated by the structure, composition, and properties of the target sequence. In general, the oligonucleotide probes range from about 6, 8, 10, 12, 15, 20, 30 to about 100 bases long, with from about 10 to about 80 bases being preferred, and from about 15 to about 40 bases being particularly preferred. That is, generally entire genes are rarely used as probes. In some embodiments, much longer nucleic acids can be used, up to hundreds of bases. The probes are sufficiently specific to hybridize to a complementary template sequence under conditions known by those of skill in the art. The number of mismatches between the probe's sequences and their complementary template (target) sequences to which they hybridize during hybridization generally do not exceed 15%, usually do not exceed 10% and preferably do not exceed 5%, as-determined by BLAST (default settings).
   Oligonucleotide probes can include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention. The sugar or glycoside portion of the probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.
   In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (e.g., U.S. Pat. No. 5,470,967) and polyamide (i.e., peptide nucleic acids). Peptide nucleic acids are described in Nielsen et al. (1991) Science 254: 1497-1500, U.S. Pat. No. 5,714,331, and Nielsen (1999) Curr. Opin. Biotechnol. 10:71-75.
   In certain embodiments, the probe can be a chimeric molecule; i.e., can comprise more than one type of base or sugar subunit, and/or the linkages can be of more than one type within the same primer. The probe can comprise a moiety to facilitate hybridization to its target sequence, as are known in the art, for example, intercalators and/or minor groove binders. Variations of the bases, sugars, and internucleoside backbone, as well as the presence of any pendant group on the probe, will be compatible with the ability of the probe to bind; in a sequence-specific fashion, with its target sequence. A large number of structural modifications, are possible within these bounds. Advantageously, the probes according to the present invention may have structural characteristics such that they allow the signal amplification, such structural characteristics being, for example, branched DNA probes as those described by Urdea et al. (Nucleic Acids Symp. Ser., 24:197-200 (1991)) or in the European Patent No. EP-0225,807. Moreover, synthetic methods for preparing the various heterocyclic bases, sugars, nucleosides and nucleotides that form the probe, and preparation of oligonucleotides of specific predetermined sequence, are well-developed and known in the art. A preferred method for oligonucleotide synthesis incorporates the teaching of U.S. Pat. No. 5,419,966.
   Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target gene are used. That is, two, three, four or more probes, are used to build in a redundancy for a particular target. The probes can be overlapping (i.e. have some sequence in common), or are specific for distinct sequences of a gene, When multiple target polynucleotides are to be detected-according to the present invention, each probe or probe group corresponding to a particular target polynucleotide is situated in a discrete area of the microarray.
   Probes may be in solution, such as in wells or on the surface of a micro-array, or attached to a solid support. Examples of solid support materials that can be used include a plastic, a ceramic, a metal, a resin, a gel and a membrane. Useful types of solid supports include plates, beads, magnetic material, microbeads, hybridization chips, membranes, crystals, ceramics and self-assembling monolayers. One example comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the chip can assist in the identification of the target nucleotide sequence. Patterns can be manually or-computer analyzed, but it is clear that positional sequencing by hybridization lends itself to computer analysis and automation. In another example, one may use an Affymetrix chip on a solid phase structural support in combination with a fluorescent bead based approach. In yet another example, one may utilise a cDNA microarray. In this regard, the oligonucleotides described by Lockkart et al (i.e. Affymetrix synthesis probes *in situ* on the solid phase) are particularly preferred, that is, photolithography.
   As will be appreciated by those in the art, nucleic acids can be attached or immobilized to a solid support in a wide variety of ways. By "immobilized" herein is meant the association or binding between the nucleic acid probe and the solid support is sufficient to be stable under the conditions of binding, washing, analysis, and removal. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as streptavidin, to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.
   Nucleic acid probes may be attached to the solid support by covalent binding such as by conjugation with a coupling agent or by covalent or non-covalent binding such as electrostatic interactions, hydrogen bonds or antibody-antigen coupling, or by combinations thereof. Typical coupling agents include biotin/avidin, biotin/streptavidin, Staphylococcus aureus protein A/IgG antibody F_{c} fragment, and streptavidin/protein A chimeras (T. Sano and C. R. Cantor, Bio/Technology 9:1378-81 (1991)), or derivatives or combinations of these agents. Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative. Derivatives which have been found to be useful include 3 or 4 [bis-(4-methoxyphenyl)]-methyl-benzoic acid, N-succinimidyl-3 or 4 [bis-(4-methoxyphenyl)]-methyl-benzoic acid, N-succinimidyl-3 or 4 [bis-(4-methoxyphenyl)]-hydroxymethyl-benzoic acid, N-succinimidyl-3 or 4 [bis-(4-methoxyphenyl)]-chloromethyl-benzoic acid, and salts of these acids.
   In general, the probes are attached to the biochip in a wide variety of ways, as will be appreciated by those in the art. As described herein, the nucleic acids can either be synthesized first, with subsequent attachment to the biochip, or can be directly synthesized on the biochip.
   The biochip comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. The solid phase support of the present invention can be of any solid materials and structures suitable for supporting nucleotide hybridization and synthesis. Preferably, the solid phase support comprises at least one substantially rigid surface on which the primers can be immobilized and the reverse transcriptase reaction performed. The substrates with which the polynucleotide microarray elements are stably associated and may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon.RTM., fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads. Each tagged bead has different primers attached to it. Tags are detectable by signalling means such as color (Luminex, Illumina) and electromagnetic field (Pharmaseq) and signals on tagged beads can even be remotely detected (e.g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.
   In one embodiment, the stirface of the biochip and the probe may be derivatized with chemical functional groups for subsequent attachment of the two. Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, for example using linkers as are known in the art; for example, homo-or heterobifunctional linkers as are well known (see 1994 Pierce Chemical Company catalog, technical section on cross-linkers, pages 155-200. In addition, in some cases, additional linkers, such as alkyl groups (including substituted and heteroalkyl groups) may be used.
   In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleoside. In an additional embodiment, the immobilization to the solid support may be very strong, yet non-covalent. For example, biotinylated oligonucleotides can be made, which bind to surfaces covalently coated with streptavidin, resulting in attachment.
   The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), U.S. Pat. No. 5,384,261 (in situ synthesis by mechanically directed flow paths); and U.S. Pat. No. 5,700,637 (synthesis by spotting, printing or coupling). Another method for coupling DNA to beads uses specific ligands attached to the end of the DNA to link to ligand-binding molecules attached to a bead. Possible ligand-binding partner pairs include biotin-avidin/streptavidin, or various antibody/antigen pairs such as digoxygenin-antidigoxygenin antibody (Smith et al, Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established, See Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994). Immobilization can be accomplished either by in situ DNA synthesis (Maskos and Southern, *supra*) or by covalent attachment of chemically synthesized oligonucleotides (Guo *et al., supra*) in combination with robotic arraying technologies.
   In addition to the solid-phase technology represented by biochip arrays, gene expression can also be quantified using liquid-phase arrays. One such system is kinetic polymerase chain reaction (PCR). Kinetic PCR allows for the simultaneous amplification and quantification of specific nucleic acid sequences, The specificity is derived from synthetic oligonucleotide primers designed to preferentially adhere to single-stranded nucleic acid sequences bracketing the target site. This pair of oligonucleotide primers form specific, non-covalently bound complexes on each strand of the target sequence. These complexes facilitate in vitro transcription of double-stranded DNA in opposite orientations. Temperature cycling of the reaction mixture creates a continuous cycle of primer binding, transcription, and re-melting of the nucleic acid to individual strands. The result is an exponential increase of the target dsDNA product. This product can be quantified in real time either through the use of an intercalating dye or a sequence specific probe. SYBR(r) Green 1, is an example of an intercalating dye, that preferentially binds to dsDNA resulting in a concomitant increase in the-fluorescent signal. Sequence specific probes, such as used with TaqMan.RTM. technology, consist of a fluorochrome and a quenching molecule covalently bound-to opposite ends of an oligonucleotide. The probe is designed to selectively bind the target DNA sequence between the two primers. When the DNA strands are synthesized during the PCR reaction, the fluorochrome is cleaved from the probe by the exonuclease activity of the polymerase resulting in signal dequenching. The probe signalling method can be more specific than the intercalating dye method, but in each case, signal strength is proportional to the dsDNA product produced. Each type of quantification method can be used in multi-well liquid phase arrays with each well representing primers and/or probes specific to nucleic acid sequences of interest. When used with messenger RNA preparations of tissues or cell lines, an array of probe/primer reactions can simultaneously quantify the expression of multiple gene products of interest. See Germer et al., Genome Res. 10:258-266 (2000); Heid et al., Genome Res. 6:986-994 (1996).
(iv) Measurement of altered location marker protein levels in cell extracts, for example by immunoassay.
   Testing for proteinaceous location marker expression product in a biological sample can be performed by any one of a number of suitable methods which are well known to those skilled in the art. Examples of suitable methods include, but are not limited to, antibody screening of tissue sections, biopsy specimens or bodily fluid samples.
   To the extent that antibody based methods of diagnosis are used, the presence of the marker protein may be determined in a number of ways such as by Western blotting, ELISA or flow cytometry procedures. These, of course, include both single-site and two-site or "sandwich" assays of the non-competitive types, as well as in the traditional competitive binding assays. These assays also include direct binding of a labelled antibody to a target.
   Sandwich assays are among the most useful and commonly used assays and are favoured for use in the present invention. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample. Variations on the forward assay include a simultaneous assay, in which both sample and labelled antibody are added simultaneously to the bound antibody. These techniques are well known to those skilled in the art, including any minor variations as will be readily apparent.
   In the typical forward sandwich assay, a first antibody having specificity for the marker or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking, covalently binding or physically adsorbing, the polymer-antibody complex is washed in preparation for the test sample. An aliquot of the sample to be tested is then added to the solid phase complex and incubated for a period of time sufficient (e.g. 2-40 minutes) and under suitable conditions (e.g. 25°C) to allow binding of any subunit present in the antibody. Following the incubation period, the antibody subunit solid phase is washed and dried and incubated with a second antibody specific for a portion of the antigen. The second antibody is linked to a reporter molecule which is used to indicate the binding of the second antibody to the antigen.
   An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.
   By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.
   In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample. "Reporter molecule" also extends to use of cell agglutination or inhibition of agglutination such as red blood cells on latex beads, and the like.
   Alternately, fluorescent compounds, such as fluorecein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest, Immunofluorescene and EIA techniques are both very well established in the art and are particularly preferred for the present method. However, other reporter molecules, such as radioisotope, chemiluminescent or bioluminescent molecules, may also be employed.
(v) Determining altered expression of protein location markers on the cell surface, for example by immunohistochemistry.
(vi) Determining altered protein expression based on any suitable functional test, enzymatic test or immunological test in addition to those detailed in points (iv) and (vi) above.

A person of ordinary skill in the art could determine, as a matter of routine procedure, the appropriateness of applying a given method to a particular type of biological sample.

Without limiting the present disclosure in any way, and as detailed above, gene expression levels can be measured by a variety of methods known in the art. For example, gene transcription or translation products can be measured. Gene transcription products, i.e., RNA, can be measured, for example, by hybridization assays, run-off assays., Northern blots, or other methods known in the art.

Hybridization assays generally involve the use of oligonucleotide probes that hybridize to the single-stranded RNA transcription products. Thus, the oligonucleotide probes are complementary to the transcribed RNA expression product. Typically, a sequence-specific probe can be directed to hybridize to RNA or cDNA. A "nucleic acid probe", as used herein, can be a DNA probe or an RNA probe that hybridizes to a complementary sequence. One of skill in the art would know how to design such a probe such that sequence specific hybridization will occur. One of skill in the art will further know how to quantify the amount of sequence specific hybridization as a measure of the amount of gene expression for the gene was transcribed to produce the specific RNA.

The hybridization sample is maintained under conditions that are sufficient to allow specific hybridization of the nucleic acid probe to a specific gene expression product. "Specific hybridization", as used herein, indicates near exact hybridization (e.g., with few if any mismatches). Specific hybridization can be performed under high stringency conditions or moderate stringency conditions. In one embodiment, the hybridization conditions for specific hybridization are high stringency. For example, certain high stringency conditions can be used to distinguish perfectly complementary nucleic acids from those of less complementarity. "High stringency conditions", "moderate stringency conditions" and "low stringency conditions" for nucleic acid hybridizations are explained on pages 2.10.1-2.10.16 and pages 6.3.1-6.3.6 in Current Protocols in Molecular Biology (Ausubel, F. et al., "Current Protocols in Molecular Biology", John Wiley & Sons, (199 8). The exact conditions that determine the stringency of hybridization depend not only on ionic strength (e.g., 0.2.times.SSC, 0.1.times.SSC), temperature (e.g., room temperature, 42°C., 68°C.) and the concentration of destabilizing agents such as formamide or denaturing agents such as SDS, but also on factors such as the length of the nucleic acid sequence, base composition, percent mismatch between hybridizing sequences and the frequency of occurrence of subsets of that sequence within other non-identical sequences. Thus, equivalent conditions can be determined by varying one or more of these parameters while maintaining a similar degree of identity or similarity between the two nucleic acid molecules. Typically, conditions are used such that sequences at least about 60%, at least about 70%, at least about 80%, at least about 90% or at least about 95% or more identical to each other remain hybridized to one another. By varying hybridization conditions from a level of stringency at which no hybridization occurs to a level at which hybridization is first observed, conditions that will allow a given sequence to hybridize (e.g., selectively) with the most complementary sequences in the sample can be determined.

Exemplary conditions that describe the determination of wash conditions for moderate or low stringency conditions are described in Kraus, M. and Aaronson, S., 1991. Methods Enzymol., 200:546-556; and in, Ausubel et al., Current Protocols in Molecular Biology, John Wiley & Sons, (1998). Washing is the step in which conditions are usually set so as to determine a minimum level of complementarity of the hybrids. Generally, starting from the lowest temperature at which only homologous hybridization occurs, each °C. by which the final wash temperature is reduced (holding SSC concentration constant) allows an increase by, 1% in the maximum mismatch percentage among the sequences that hybridize. Generally, doubling the concentration of SSC results in an increase in Tₘ of about 17°C. Using these guidelines, the wash temperature can be determined empirically for high, moderate or low stringency, depending on the level of mismatch sought. For example, a low stringency wash can comprise washing in a solution containing 0,2.times.SSC/0.1% SDS for 1 minutes at room temperature; a moderate stringency wash can comprise washing in a pre-warmed solution (42°C.) solution containing 0.2.times.SSC/0.1% SDS for 15 minutes at 42°C.; and a high strigency wash can comprise washing in pre-warmed (68°C.) solution containing 0.1.times.SSC/0.1% SDS for 15 minutes at 68°C. Furthemore, washes can be performed repeatedly or sequentially to obtain a desired result as known in the art. Equivalent conditions can be determined by varying one or more of the parameters given as an example, as known in the art, while maintaining a similar degree of complementarity between the target nucleic acid molecule and the primer or probe used (e.g., the sequence to be hybridized).

Also described is a nucleic acid array, which array comprises a plurality of:
(i) nucleic acid molecules comprising a nucleotide sequence corresponding to any one of the location marker genes hereinbefore described or a sequence exhibiting at least 80% identity thereto or a functional derivative, fragment, variant or homologue of said nucleic acid molecules; or
(ii) nucleic acid molecules comprising a nucleotide sequence capable of hybridising to any one or more of the sequences of (i) under low stringency conditions at 42°C or a functional derivative, fragment, variant or homologue of said nucleic acid molecule
(iii) nucleic acid probes or oligonucleotides comprising a nucleotide sequence capable of hybridising to any one or more of the sequences of (i) under low stringency conditons at 42°C or a functional derivative, fragment, variant or homologue of said nucleic acid molecule
(iv) proteins encoded by the nucleic acid molecules of (i) or (ii) or a derivative, fragment or, homologue thereof
   wherein the level of expression of said nucleic acid is indicative of the proximal-distal origin of a cell or cellular subpopulation derived from the large intestine.

Reference herein to a low stringency at 42°C includes and encompasses from at least about 1% v/v to at least about 15% v/v formamide and from at least about 1M to at least about 2M salt for hybridisation, and at least about 1M to at least about 2M salt for washing conditions. Alternative stringency conditions may be applied where necessary, such as medium stringency, which includes and encompasses from at least about 16% v/v at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01M to at least about 0.15M salt for hybridisation, and at least about 0.01M to at least about 0.15M salt for washing conditions. In general, washing is carried out at Tₘ = 69.3 + 0.41 (G + C) % [19] = -12°C. However, the Tₘ of a duplex DNA decreases by 1°C with every increase of 1% in the number of mismatched based pairs (Bonner et al (1973) J Mol. Biol. 81:123).

A library or array of nucleic acid or protein markers provides rich and highly valuable information. Further, two or more arrays or profiles (information obtained from use of an array) of such sequences are useful tools for comparing a test set of results with a reference, such as another sample or stored calibrator. In using an array, individual nucleic acid members typically are immobilized at separate locations and allowed to react for binding reactions. Primers associated with assembled sets of markers are useful for either preparing libraries of sequences or directly detecting markers from other biological samples.

A library (or array, when referring to physically separated nucleic acids corresponding to at least some sequences in a library) of gene markers exhibits highly desirable properties. These properties are associated with specific conditions, and may be characterized as regulatory profiles. A profile, as termed here refers to a set of members that provides diagnostic information of the tissue from which the markers were originally derived. A profile in many instances comprises a series of spots on an array made from deposited sequences.

A characteristic patient profile is generally prepared by use of an array. An array profile may be compared with one or more other array profiles or other reference profiles. The comparative results can provide rich information pertaining to disease states, developmental state, receptiveness to therapy and other information about the patient.

Another aspect of the present disclosure provides a diagnostic kit for assaying biological samples comprising an agent for detecting one or more proximal-distal markers and reagents useful for facilitating the detection by the agent in the first compartment. Further means may also be included, for example, to receive a biological sample. The agent may be any suitable detecting molecule.

The present invention is described by the following non-limiting examples:

### EXAMPLE 1

### MAP OF DIFFERENTIAL TRANSCRIPT EXPRESSION IN THE NORMAL LARGE INTESTINE

### MATERIALS AND METHODS

### Gene Expression Data

To explore variation of human gene expression along the non-neoplastic large intestine, we used gene expression data collected using the Affymetrix (Santa Clara, CA USA) GeneChip®oligonucleotide microarray system described in [Lipshutz et al., 1999, Nat Genet 21:20-24]. The data are two independent Affymetrix (Santa Clara, CA USA) Human Genome 133 GeneChip datasets: a large commercial microarray database of HGU-133 A&B chip data for 'discovery', and a smaller HGU-133 Plus 2.0 microarray data set generated by us for 'validation'.

The larger data set was analyzed to identify gene expression patterns and the independently derived second expression set was used to validate these patterns. Thus, the first data set was mined for hypothesis *generation* while the second set was used for hypothesis *testing.* The data for this study are oligonucleotide microarrays hybridized to labelled cRNA synthesized from poly-A mRNA transcripts isolated from colorectal tissue specimens. The Affymetrix platform that we use is designed to quantify target mRNA transcripts using a panel of 11 perfect match 25bp oligonucleotide probes (and 11 mismatch probes), called a probeset. To determine the biological relevance of probeset binding intensity, we have annotated the resulting probeset lists using the most current Affymetrix metafiles and BioConductor libraries available. We note that there are multiple probesets on the microarray platform theoretically reactive to any given target 'gene'. As our focus is to explore transcript expression dynamics along the large intestine, and not to elucidate the underlying genomic mechanisms, we do not explore this phenomenon further. Nevertheless, this fundamental annotation detail should be considered when interpreting the biological relevance of these data and we caution the reader (and other researchers using these techniques) to be wary of the dangers of using the terms 'genes' and 'probeset' interchangeably.

### 'Discovery' data set

Gene expression and clinical descriptions for 184 colorectal tissue specimens were purchased from GeneLogic Inc. (Gaithersburg, MD, USA).

Individual tissue microarray data were selected with the following characteristics: non-neoplastic colorectal mucosa (confirmed by histology) from otherwise healthy tissue specimen (i.e. no evidence of inflammation or other disease at specimen site) with an anatomically-identifiable site of resection designated as one of: cecum, ascending colon, descending colon, sigmoid colon, or rectum.

For each tissue selected from the GeneLogic database, we received electronic files of raw data containing a total of 44,928 probesets (HGU133A and HGU133B, combined), experimental and clinical descriptors for each tissue, and digitally archived microscopy images of the histology preparations. Each data record was manually assessed for clinical consistency and a sample of records was randomly chosen for histopathology audit using digitally archived histology images. A quality control analysis was performed to identify and remove arrays not meeting essential quality control measures as defined by the manufacturer. [Affymetrix, 2001; Wilson and Miller, 2005, Bioinformatics].

Gene expression levels were calculated by both Microarray Suite (MAS) 5.0 (Affymetrix) and the Robust Multichip Average (RMA) normalization techniques. [Affymetrix, 2001; Hubbell et al, 2002, Bioinformatics 18:1585-1592; Irizarry et al., 2003, Nucleic Acids Res 31:e15] MAS normalized data was used for performing standard quality control routines and the final data set was normalized with RMA for all subsequent analyses.

### 'Validation' Data Set

The colorectal specimens in the 'validation' set were collected from a tertiary referral hospital tissue bank in metropolitan Adelaide (Repatriation General Hospital and Flinders Medical Centre). The tissue bank and this project were approved by the Research and Ethics Committee of the Repatriation-General Hospital and patient consent was received for each tissue studied. Following surgical resection, specimens were placed in a sterile receptacle and collected from theatre. The time from operative resection to collection from theatre was variable but not more than 30 minutes. Samples, approximately 125mm³ (5x5x5mm) in size, were taken from the macroscopically normal tissue as far from pathology as possible, defined both by colonic region as well as by distance either proximal or distal to the pathology. Tissues were placed in cryovials, then immediately immersed in liquid nitrogen and stored at -150°C until processing.

Frozen samples were processed by the authors using standard protocols and commercially available kits. Briefly, frozen tissues were homogenized using a carbide bead mill (Mixer Mill MM 300, Qiagen, Melbourne, Australia) in the presence of chilled Promega SV RNA Lysis Buffer (Promega, Sydney, Australia) to neutralize RNase activity. Homogenized tissue lysates for each tissue were aliquoted to convenient volumes and stored -80°C. Total RNA was extracted from tissue lysates using the Promega SV Total RNA system according to manufacturer's instructions and integrity was assessed visually by gel electrophoresis.

To measure relative expression of mRNA transcripts, tissue RNA samples were analyzed using Affymetrix HG U133 Plus 2.0 GeneChips (Affymetrix, Santa Clara, CA USA) according to the manufacturer's protocols [Affymetrix, 2004]. Biotin labelled cRNA was prepared using 5µg (1.0 µg/µL) total RNA (approx. 1 µg mRNA) with the "One-Cycle cDNA" kit (incorporating a T7-oligo(dT) primer) and the GeneChip IVT labelling kit. *In vitro* transcribed cRNA was fragmented (20 µg) and analyzed for quality control purposes by spectrophotometry and gel electrophoresis prior to hybridization. Finally, an hybridization cocktail was prepared with 15µg of cRNA (0.5 µg/µL) and hybridized to HG U133 Plus 2.0 microarrays for 16h at 45°C in an Affymetrix Hybridization Chamber 640. Each cRNA sample was spiked with standard eukaryotic hybridization controls for quality monitoring.

Hybridized microarrays were stained with streptavidin phycoerytherin and washed with a solution containing biotinylated anti-streptavidin antibodies using the Affymetrix Fluidics Station 450. Finally, the stained and washed microarrays were scanned with the Affymetrix Scanner 3000.

The Affymetrix software package was used to transform raw microarray image files to digitized format. As for the Discovery set above, gene expression levels for the validation data set were generated using MAS 5.0 (Affymetrix) for quality control purposes and with the RMA normalization method for expression data.

### Statistical analysis

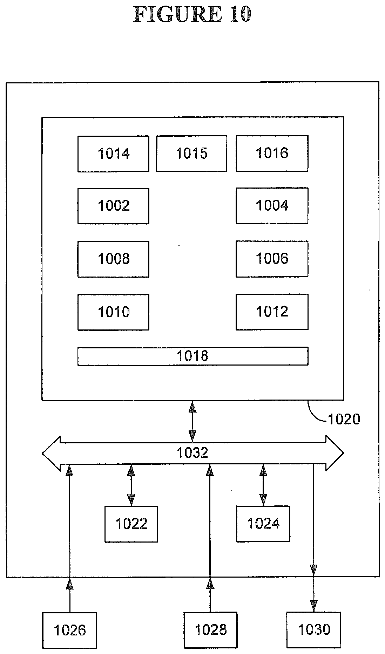

As shown in Figure 10, a detection system includes detection modules 1002 to 1007, including a support vector machine (SVM) module 1002, a profile analyser 1004, a principal component analyser 1006, and a classifier module 1007. The detection system executes detection methods that generate location data representative of the origin along the proximal-distal axis of the large intestine of a cell, or cell population, from that intestine. The location data is generated by processing gene expression data representing the expression of genes within that cell or cell population. In the described embodiment, the detection system is a standard computer system such as an Intel IA-32 based computer system, and the detection modules 1002 to 1007 are implemented as software modules stored on non-volatile (e.g., hard disk) storage 1020 associated with the computer system. However, it will be apparent that at least parts of the detection modules 1002 to 1007 or the detection methods described herein could alternatively be implemented as one or more dedicated hardware components, such as application-specific integrated circuits (ASICs). In the described embodiment, the detection system also includes C++ modules 1008 to provide C++ language support, including C++ libraries, and an R module 1012 providing support for the R statistical programming language and the MASS library described in [Venables and Ripley, 2002] and available from the CRAN open source depository at http://cran.r-project.org. The system also includes the BioConductor software application 1010 available from http://wvw.bioconductor.org, which, together with the profile analyser 1004 and principal component analyser 1006, are implemented in the R programming language, as described at http://www.r-project.org. The SVM 1002 is implemented in the C++ programming language. The classifier module 1007 is the GeneRave application, as described at http://www.bioinformatics.csiro.au/products.shtml and references provided therein. The system also includes the Microarray Suite (MAS) 5.0 1014, and the Robust Multichip Average (RMA) normalisation application 1016, both available from Affymetrix,-and described at http://www.affymetrix.com. The software applications are executed under control of a standard operating system 1018, such as Linux or Mac0S 10.4, and the computer system includes standard computer hardware components, including at least one processor 1022, random access memory 1024, a keyboard 1026, a standard pointing device such as a mouse 1028, and a display 1030, all of which are interconnected via a system bus 1032, as shown.

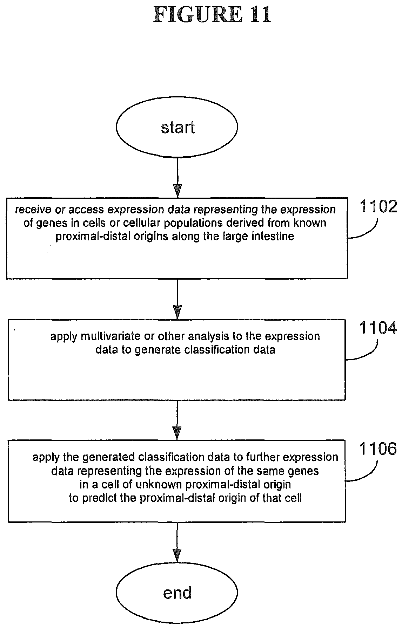

The detection methods include classification methods of the general form of Figure 11. First, at step 1102, the system receives or otherwise accesses expression data representing the expression of genes in cells of known proximal distal origin. At step 1104, a multivariate or other form of classification or decision method is applied to the expression data to generate classification data, as described below. Typically, the expression data represents the expression of genes which, either alone or in combination, are already known to be differentially expressed along the proximal- distal axis of the large intestine. However, the method can also be used to identify such genes and/or gene combinations, as described below. At step 1106, the classification data is applied to further expression data representing the expression of the same genes in a cell of unknown origin to predict the proximal-distal origin of that cell along the large intestine.

Furthermore, it will be apparent to those skilled in the art that the resulting classifier or discriminating function represented by the initially generated classification data can be adjusted based on decision theoretic principles to improve the classification outcomes and their utility. For example, a prior belief in the probability of outcomes can be incorporated, and/or a decision surface can be modified based on the different costs of misclassification cases. These and other relevant methods of decision theory, minimizing loss functions, and cost of misclassification are described in [Krzanowski and Marriott, 1995].

For all statistical analysis, we used open source software available from BioConductor for the R statistics environment (R being an open source implementation of the S statistical analysis environment). (Bioconductor, www.bioconductor.org) [Gautier et al., 2004, Bioinformatics 20:307-315; Gentleman et al., 2004, Genome Biol 5:R80].

The linear methods used to generate and process linear and non-linear combinations of' gene expression levels, including linear regression, multiple linear regression, linear discriminant analysis, logistic regression, generalized linear models, and principal components analysis, are all described in [Hastie, 2001], for example. These methods are implemented in R.

Gene expression gradients were analyzed using three analytical techniques. First, we compared the gene expression variation of individual genes along the large intestine in the usual univariate manner. Next, we further explored those particular genes exhibiting statistically significant expression differences with linear models to compare dichotomous (proximal vs. distal) expression change with a gradual (multi-segment) model of change. Finally, we applied multivariate techniques to understand subtle genome-wide expression variance along the proximal-distal axis. Such genome-wide expression variances were interrogated using non-parametric methods as described in [Ripley, 1996], including nearest neighbor methods.

### Individual gene expression maps

### Univariate differential expression

Differentially expressed gene transcripts between the proximal and distal large intestine were identified using a moderated t-test implemented in the 'limma' Bioconductor library for R [Smyth, 2005]. Significance estimates (p-values) were corrected to adjust for multiple hypothesis testing (MHT) using the conservative Bonferroni correction. The subset of tissues limited to the cecum vs. the rectum were similarly tested.

Gene transcripts identified to be differentially expressed were also evaluated in the 'Validation' specimens on a probeset-by-probeset basis using modified *t*-tests. To assess the significance of the total number of differential probesets that were likewise differential in the validation data, the number of 'validated' probesets were compared to a null distribution estimated using a Monte Carlo simulation.

### Multi-segment large intestine vs. two-segment large intestine model comparison

To evaluate the nature of inter-segment gene expression variation we analyzed differentially expressed probesets for relative fit to linear models in a multi-segment vs. a two segment framework. The goal of this analysis is to explore whether the intersegment expression of probesets that are known to be differentially expressed between the terminal ends of the large intestine are better modelled by a five-segment linear model that approximates a continual gradation or by a simpler, dichotomous 'proximal' vs. 'distal' gradient. As our data are only identified by colorectal segment designation and not by a continuous measurement along the length of the large intestine, we approximate the continuous model using the tissue segment location. We chose probesets that are differentially expressed between the most terminal segments (cecum and rectum) in order to maximize the likelihood of identifying transcripts that vary along the proximal-distal axis of the large intestine.

We first modelled the expression of these probesets along the proximal-distal axis of the large intestine using a five factor robust linear model according to an indicator matrix defined by the colorectal segment for each tissue. For this model each tissue was assigned by biopsy location to one of: cecum, ascending, descending, sigmoid, or rectum. (For reasons described below, transverse tissues were not included in this analysis.) This five segment model was then compared to a two-factor robust linear model with a design matrix corresponding to the theoretical proximal and distal regions of the large intestine. The same data were used for both model comparisons, however for the two segment model, the first factor (corresponding to the proximal tissues) included all of the tissues from the cecum and ascending colon while the second factor (corresponding to the distal large intestine) included all tissues from the descending, sigmoid and rectum segments.

When comparing these distinct models for each probeset, we used an *F*-test to evaluate the hypothesis Ha that the improved fit (reduced regression residual) provided by the more complex five-segment model was significantly better than the simpler two segment model. A non-significant residual reduction indicates a failure to reject the null hypothesis
H0: that there is no inherent value to adopting a more complex five segment model over the simpler alternative.

### Multivariate gene expression pattern mapping

### RESULTS

### Gene expression data collection

### Discovery and Validation Data Sets

A discovery data set was generated using data from the hybridization of cRNA to Affymetrix HG U133A/B GeneChip microarrays that were purchased from GeneLogic Inc.

Data from 184 normal tissues meeting inclusion criteria and quality assurance criteria for the HG U133A/B GeneChip were analyzed and used for hypothesis generation. The tissues comprised segment subsets as follows: 29 cecum, 45 ascending, 13 descending, 54 sigmoid, and 43 rectum. For each tissue, 44,928 probe sets were background corrected and normalized using RMA pre-processing.

To construct the 'validation' data set, 19 HG U133 Plus2.0 GeneChips were hybridized to labelled cRNA prepared from 8 proximal tissue specimens and 11 distal specimens. Due to stringent quality control parameters for tissue and GeneChip acceptability, this validation data set did not include sufficient tissues to explore multiple segment models. Each microarray measured transcript expression for 54,675 probe sets.

The theoretical juncture between the proximal and distal large intestine is approximately two thirds the length of the transverse colon measured from the hepatic flexure. [Yamada and Alpers, 2003, *supra*] As sample data were not specific for distance along the transverse colon, these tissues were excluded from the discovery analysis.

### Gene variation along the large intestine

### Individual gene expression changes

### Univariate differential expression

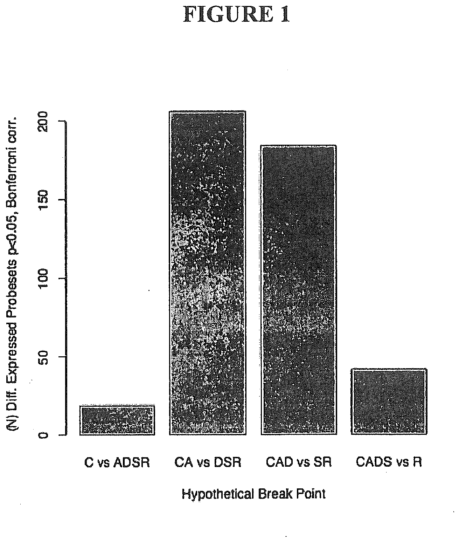

To explore the 'natural' dividing point between the anatomical segments of the large intestine, we measured the absolute number of probeset expression changes when the hypothetical 'divide' was moved stepwise from cecum to rectum. Figure 1 shows the number of probesets that were differentially expressed for all continuous inter-segment combinations. While not statistically significant, the maximum number of probeset differences, 206, occurs when the proximal and distal regions are divided between the ascending and descending segments. As this dividing point is consistent with both our understanding of embryonic development and the usual separation of the proximal and distal segments, our work assumes that the proximal and distal tissues are separated in this fashion.

A total of 206 probesets, corresponding to approximately 154 known gene targets, were differentially expressed higher in the proximal or distal colorectal samples compared to the corresponding region (Bonferroni corrected p<0.05). Of these 206 probesets, 31 (16.5 %) were also differentially expressed in the validation data with a significant difference (31/206, p << 0.05 by Monte Carlo estimation).

A total of 115 probesets were differentially expressed between tissues selected only from the cecum (n=29) and the rectum (n=43). While 102 (89%) of these probesets are included in the 206 probesets differing between proximal and distal large intestine described above, the cecum vs. rectum gene expression is useful, in principle, to isolate those transcripts that are different between the most terminal ends of the large bowel. In this subset, 28 probesets (24.3 %) were likewise differentially expressed in the rectum vs. the cecum in the validation data (28/115, p<<10-5 by Monte Carlo estimation).

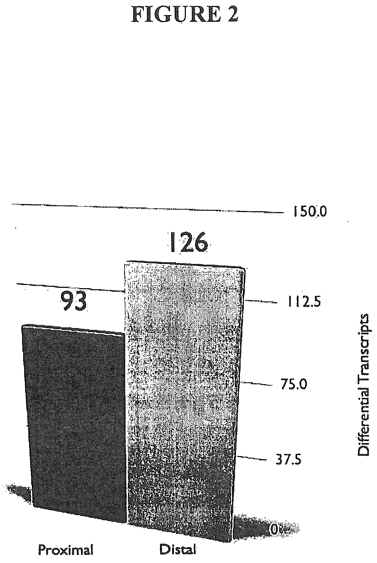

Differentially expressed probesets and difference statistics for probesets with elevated expression in proximal and distal tissues are shown in Tables 1 and 2, respectively. Figure 2 compares the number of probesets expressed significantly higher in the proximal (n=94) or distal (n=126) gut (or cecum and rectum), respectively.

### Multi-segment gene expression models

An analysis for differential expression was also made for all five inter-segment transitions in order from the cecum to the rectum (i.e. cecum vs. ascending, ascending vs. transverse, etc.). Interestingly, no transcript was differentially expressed to a significant degree between any two adjoining segments (moderated *t*-test; p < 0.05).

To explore the precise nature of these gene transcript expression changes, we built and compared robust linear models fitted to the expression data based on location for each tissue sample. Two robust linear models of univariate probeset expression were compared for each of the 115 probesets differentially expressed between the two terminal segments of the large intestine, the cecum and rectum. In particular, we queried whether the expression of those transcripts that were differentially expressed between these terminal segments were better explained (in terms of residual fit) by a simple two-segment model or by the more descriptive five-segment model.

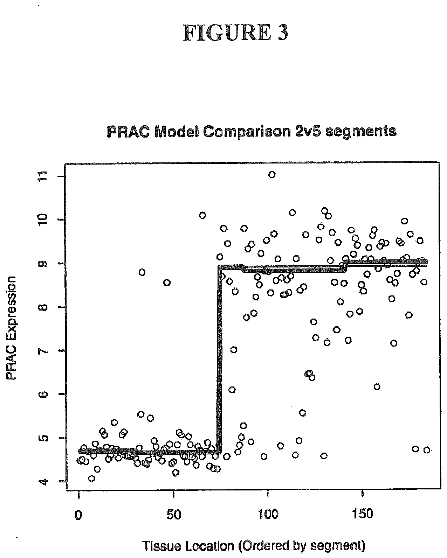

Of the 115 differentially expressed probesets, the analysis failed to reject the null hypothesis that a complex model does not significantly improve model fit to the observed gene expression data for 65 (57%) of cases (F-test, p > 0.05). Thus, more than half of these differentially expressed transcripts along the large intestine are satisfactorily modelled by the two segment expression model whereby expression is dichotomous and defined by either proximal vs. distal location. The most differentially expressed probeset between the cecum and rectum is the transcript for *PRAC.* A comparison of the two-segment and multi-segment models for this transcript are shown in Figure 3.

For the remaining 50 (43%) probesets, the null hypothesis was rejected (p<0.05), suggesting that a five factor model dependent on segment location in fact improves the predictive effectiveness of such transcripts' expression along the proximal-distal axis in a significant manner. Inspection of these models confirms that most models are monotonic increasing or monotonic decreasing in tissues progressing along the large intestine.

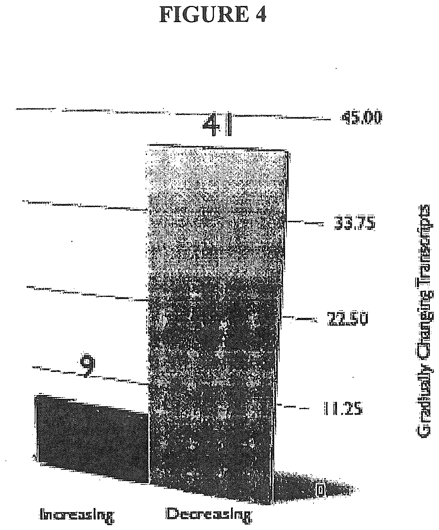

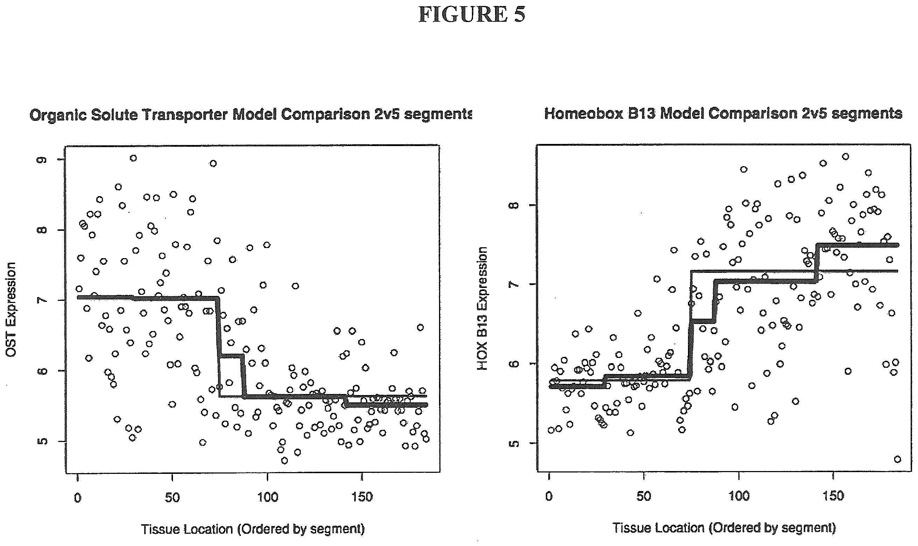

Interestingly, 41 (82%) of the 50 multi-segment models show a gradual *increase* across the large intestine while only 9 models (18%) indicate a gradual *decrease* from proximal to distal expression (shown in Figure 4). The models for both the organic solute transporter, alpha (*OSTalpha*) and homeobox gene B13 (*HOXB13*) are significantly improved with the five segment model as illustrated in Figure 5.

### Patterns of gene expression along the large intestine

In addition to analyses of individual gene changes along the large intestine, we used multivariate analytical techniques to explore patterns of gene changes along the proximal-distal axis.

### Supervised Principal Components Analysis

To visualize and explore the structure of expression variability at an organ level, principal component analysis (PCA) and a variant of PCA known as Supervised PCA were applied to the gene expression data using the principal component analyzer (PCA) 1006 of the detection system. PCA is described in [Venables and Ripley, 2002], and was implemented in R. A detailed description of supervised PCA can be found in [Bair *et al.,* 2004].

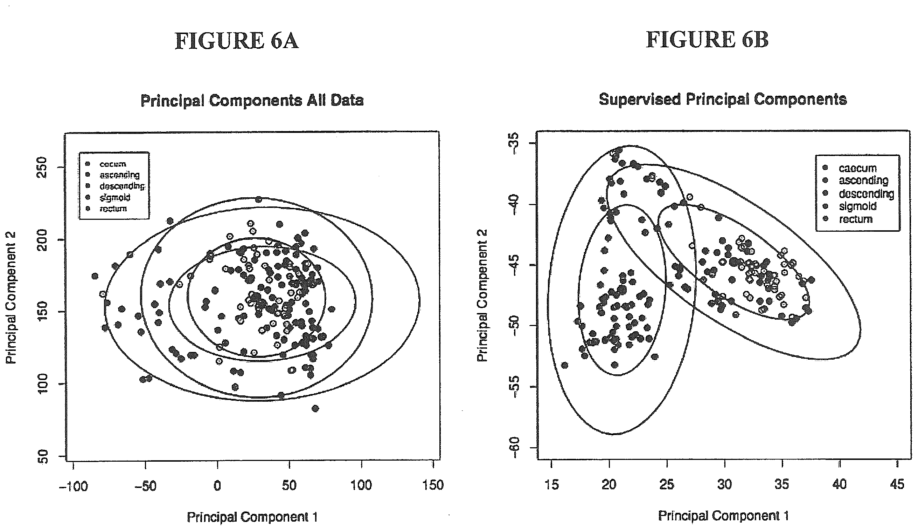

Initially, expression data representing gene expression of all 44,928 probesets of the 'Discovery' data set were processed by the PCA module 1006 using principal components analysis (PCA). PCA is a standard method for simplifying a multi-dimensional data set by generating linear transformations of the data set dimensions to reduce the number of dimensions. The transformed data is provided as principal component data representing a sorted set of "principal components", such that the first principal component has the greatest variance, the second principal component the second greatest variance, and so on. The result of applying PCA to the complete data set includes the multivariate or principal component data shown in Figure 6A, which is a graph in which the first principal component is plotted on the x-axis, and the second principal component on the y-axis. Inspection of this low dimension perspective yield no obvious structure within the data that is consistent with tissue segment, suggesting that the major sources of gene expression variation measured across all genes is independent of tissue location.

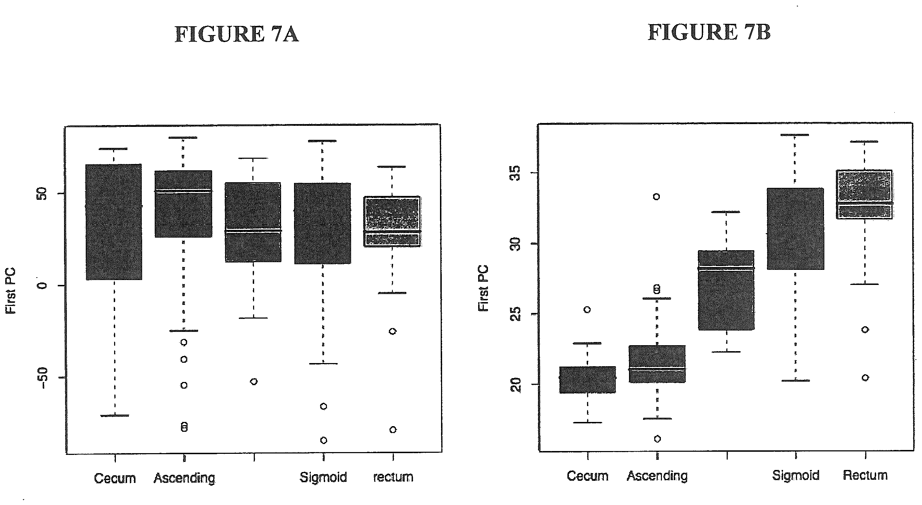

To investigate whether a subset of all genes could be used to generate one or more principal components indicative of tissue location, the expression data was analysed by supervised PCA. As described in [Bair et al, 2004], supervised PCA is similar to standard principal components analysis but uses only a subset of the features/genes (usually selected by some univariate means) to generate the principal components. In this case, the set of genes differentially expressed between the cecum and rectum (i.e., the extreme ends of the large intestine) were selected for PCA analysis. However, other forms of feature selection could alternatively be used. Specifically, a reduced data matrix was generated by including only the 115 probesets that are differentially expressed between tissue samples taken from the cecum and rectum, but for all 184 normal tissues from all segments of the large intestine. Standard PCA was then performed on this feature specific data. As shown in Figure 6B, a graph of the first two principal components suggests the existence of two broad sub-populations within the 184 tissue samples, corresponding approximately to the proximal vs. distal divide. This dependence on cell origin is visualised more clearly if the first principal component is graphed as a function of cell origin along the large intestine, as shown in Figure 7B. The symbols in Figure 7B represent the interquartile range (i.e. half the data) and the "error bars" indicate 1.5 x the interquartile range. Data outside these limits are considered to be outliers and are plotted individually. While there is perhaps the suggestion of a weak separation between the sigmoid colon and rectum, the anterior tissues of cecum and ascending colon strongly overlap with poor separation.

Although the principal component data could be used to predict the origin of cells based on expression of genes from these cells, other analysis methods are preferred for this task, as described below.

### Profile Analysis (Canonical variate analysis)

Expression patterns along the gut were also analyzed by the profile analyser 1004 using Profile Analysis to visualize inter versus intra-segment expression variation. As described in [Kiiveri, 1992], profile analysis is a modification of standard canonical variate analysis suited to cases where the number of variables exceeds the number of observations. The method models the *p x p* within-class covariance matrix ∑*_{w}* via a factor analytic model [Kiiveri, 1992] with a relatively low number of independent factors. Permutation tests are used to determine the significance of each term (i.e. gene) in each of the canonical variates. By including only significant terms, profile analysis provides a feature selection capability. This method is generally useful as an exploratory tool to characterize the class variation structure. Canonical variate analysis is implemented in the R MASS library, as described in [Venables and Ripley, 2002]. Profile Analysis was implemented in a proprietary library in R, as described in [Kiiveri 1992].

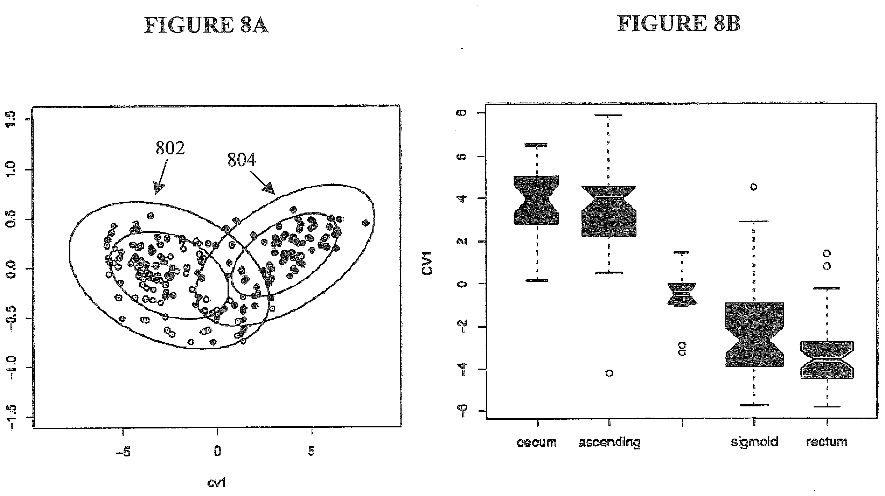

Given *a priori* knowledge of segment labels for tissues, profile analysis attempts to identify the limited gene transcript subspace that provides maximum inter-class separation of each of the five segments of the large intestine while minimizing the intraclass (i.e., with each segment) variance. The results of profile analysis of the complete data set include the canonical variable data shown in Figure 8A, as a graph wherein the first canonical variate is plotted along the x-axis, and the second canonical variate along the y-axis. It is apparent that the tissue segments correlate with the first canonical variate, but the second and subsequent canonical variates provide little or no class separation information. This result suggests that the same probesets are involved in separating each of the colorectal segments, i.e., the largest sources of difference from-a tissue-segment perspective are those used to generate the first canonical variate dimension and hence all of the segments are best grouped by this same feature set of probesets. As shown in Figure 8B, even when the first canonical variate is used, none of the segments is perfectly separated, although the natural ordering of the segments is clearly preserved. As with PCA described above, the canonical variate data could be used to classify the proximal-distal origin of cells at unknown origin, but the methods described below are preferred for this purpose.

### Support Vector Machines

While the multivariate methods described above are useful for investigating gene expression variation along the large intestine, supervised machine learning was used to identify genes that are also *predictive* of tissue location in a robust manner, and to identify the smallest subsets of probesets/genes that can be used to predict tissue location with a low-cross validated error rate.

In the described embodiment, the particular form of machine learning used is a support vector machine (SVM), as provided by the SVM module 1002; however, it will be apparent to the skilled addressee that other kernel methods could alternatively be used. As described in [Scholkopf, 2004], kernel methods are extensions of linear methods whereby the variables are mapped to another space where the essential features of this mapping are captured by a simple kernel. Kernel methods can be particularly advantageous in cases where the observations are linearly separable in the kernel space but not in the original data space.

The SVM 1002 determines the combination of features (gene transcripts) that maximally separates the observations (i.e., tissues) along a class-decision boundary, using standard SVM methodology, as described in [Cristianini and Shawe-Taylor, 2000].

Specifically, the support vector machine (SVM) 1002 was used to generate classification data representing the smallest sub-set of probesets from the complete data set whose expression enables the maximum separation of cells originating from the cecum and rectum. The SVM 1002 was trained using a linear kernel and the classification data generated at each iteration was evaluated using 10-fold cross-validation. The lowest contributing gene transcripts from each subset of transcripts were recursively eliminated to identify the smallest set of transcripts with high prediction accuracy.

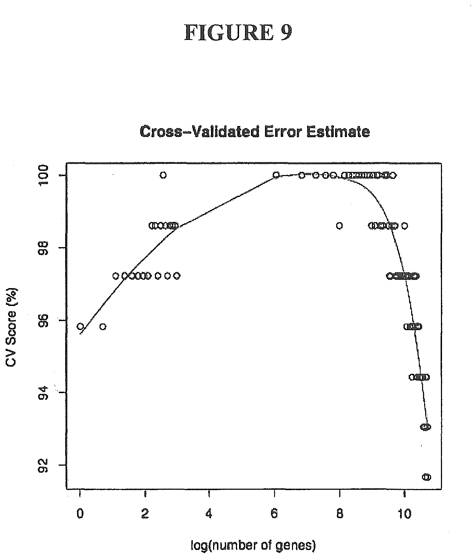

The cross-validated SVM error rate as a function of the number of probesets included in the model (as they were successively eliminated) is shown in Figure 9. The smallest feature set that yields a perfect (0%) cross-validated error rate includes the 13 probesets shown in Table 3.

To measure the utility of this model in an independent data set, the classification data for the thirteen feature model was tested for proximal vs. distal prediction performance in the validation data. Using a traditional linear discriminant analysis model built with these 13 probesets, the eight proximal and eleven distal tissues were predicted with 100% accuracy.

### Classifier Model

As an alternative to the SVM 1002, a classifier 1007 was also used to process the complete expression data from tissue samples taken from known locations along the proximal-distal axis of the large intestine to identify combinations of genes that can be used to identify the origin of a cell or cell population of unknown origin along the large intestine. In the described embodiment, the linear GeneRave classifier was used, as described at http://www.bioinformatics.csiro.au/overview.shtml. GeneRave is preferred in cases where the number of variables exceeds the number of observations. However, it will be apparent to those skilled in the art that other classifiers could be alternatively used, including non-linear classifiers and classifiers based on regularized logistic regression.

As described in [Kiiveri 2002], the GeneRave classifier 1007 generates classification data representing linear combinations of expression levels to identify subsets of genes that can be used to accurately identify the location of a sample of unknown location. GeneRave 1007 uses a Bayesian network model to select genes by eliminating genes that in linear combination with other genes do not have any correlation with the location from which corresponding tissue samples were taken.

The result of the GeneRave analysis of the complete data set in classification data corresponding to a set of 7 genes whose expression levels can be used to accurately identify the origin of a corresponding cell along the proximal-distal axis of the large intestine. The 7 genes are SEC6L1, PRAC, SPINK5, SEC6L1, ANPEP, DEFA5, and CLDN8.

### Discussion

### A map of gene differential expression along the large intestine

Univariate expression analysis identified 206 probesets corresponding to 154 unique gene targets that are differentially expressed between the normal proximal and normal distal large intestine regions in human adults. A subset of 115 probesets (89% common to the proximal vs. distal list) is likewise differentially expressed between the terminal colorectal segments of the cecum and rectum. Interestingly, we found no transcripts that were expressed significantly differently between any two adjacent segments.

To estimate the validity of these findings, we have also measured the expression change of these gene transcripts in an independent set of microarray data. Thirty-one (31) of the 206 differentially expressed probesets in our initial discovery data set of 184 colorectal tissue samples were also differentially expressed in the validation data of 19 specimens.

Using a Monte Carlo simulation, we showed that such a large number of probesets differential in both datasets is extremely unlikely.

Nearly all (28/31, 90%) of these 'validated' transcripts were likewise differentially expressed between the two terminal segments of the cecum and rectum. 57 of 154 (37%) corresponding gene targets were confirmed to be differentially expressed between the proximal and distal large intestine by independent means.

### Differential transcript expression for individual genes

The most significantly differential probeset we observed in our discovery data was against the gene transcript for *PRAC. PRAC* is highly expressed in the distal large intestine relative to the proximal tissues. Further, *PRAC* appears to be expressed in a low-high pattern along the large intestine with a sharp expression change occurring between the ascending and descending colorectal specimens.

We found eight (8) probesets corresponding to seven (7) *HOX* genes to be differentially expressed between the proximal and distal large intestine. The 39 members of the mammalian homeobox gene family consist of highly conserved transcription factors that specify the identity of body segments along the anterior-posterior axis of the developing embryo[Hostikka and Capecchi, 1998, Mech Dev 70:133-145; Kosaki et al., 2002, Teratology 65:50-62]. The four groups of *HOX* gene paralogues are expressed in an anterior to posterior sequence, for e.g. from *HOXA1* to *HOX13.* [Montgomery et al., 1999, Gastroenterology 116:702-731] It has been found that: lower numbered *HOX* genes are expressed higher in the proximal tissues (*HOXD3, HOXD4, HOXB6, HOXC6* and *HOXA9*), while the higher named genes are more expressed in the distal large intestine (*HOXB13* and *HOXD13*).

Interestingly, there was a conspicuous absence in our findings of some gene transcripts that have been previously shown to be differentially expressed along the proximal-distal axis. Our data do not demonstrate a significant expression gradient for the caudal homeobox genes *CDX1* or *CDX2,* transcription factors that have been shown to be involved in intestine pattern development across a range of vertebrates. (Chalmers *et al.,* 2000) (James *et al.,* 1994) (Silberg *et al.,* 2000) In particular, *CDX2* is believed to play a role in maintaining the colonic phenotype in the adult large intestine and was recently shown to be present at relatively high concentrations in the proximal large intestine but absent in the distal large intestine (James *et al.,* 1994) (Silberg *et al.,* 2000). Neither statistical analysis nor visual inspection of probeset expression for this gene show differential expression along the large intestine in our data (data not shown).

We observed significant differential transcript expression for a number of the solute-carrier transport genes. While probeset expression for *SLC2A10, SLC13A2,* and *SLC28A2* are higher in the distal large intestine, the solute carrier family members *SLC9A3, SLC14A2, SLC16A1, SLC20A1, SCL23A3,* and *SLC37A2* are higher in the proximal tissues.

Our results show that probesets against all three of the five members of the chromosome 7q22 cluster of membrane-bound mucins previously believed to be expressed in large intestine, *MUC11, MUC12* and *MUC17,* are differentially expressed at higher levels in the distal gut [Byrd and Bresalier, 2004, Cancer Metastasis Rev 23:77-99; Williams et al., 1999, Cancer Res 59:4083-4089; Gum et al., 2002, Biochem Biophys Res Commun 291:466-475]. We also confirmed this differential expression pattern for *MUC12* and *MUC17* in the independent validation data. Previous reports have also raised the question about whether the genomic sequences for MUC11 and *MUC12* are from closely related or perhaps even the same gene. [Byrd and Bresalier, 2004, *supra*] Correlation analysis of *MUC11* and *MUC12* probesets show a strong, positive correlation at the lower end of the probeset expression range with a weaker correlation as expression increases (data not shown). This correlation profile could be due to increased variability at higher expression levels or, possibly, because the expression levels in the distal large intestine (where they are higher) reflect a distinct transcriptional control.

In addition, while previous research has suggested that the secreted, gel-forming mucin *MUC5B* is only weakly expressed in the large intestine[Byrd and Bresalier, 2004, *supra*], our results show that probesets reactive to this transcript are expressed higher in the distal large intestine as for the membrane-bound mucins.

Some of the expression patterns we report here for humans have been shown to be similarly patterned in the gastrointestinal tracts of rodent models. However, a number of specific genes previously shown to be differentially expressed along the large intestines of mice and rats were not found to be so expressed by us. Such gene transcript targets include, carbonic anhydrase IV (Fleming *et al.,* 1995), solute carrier family 4 member 1 (alias *AE1*) (Rajendran *et al.,* 2000), CD36/fatty acid translocase (Chen *et al.,* 2001), and toll-like receptor 4 (Ortega-Cava *et al.,* 2003). On the other hand, our data are in agreement with earlier studies of expression of aquaporin-8 (*AQP8*), a gene whose expression product is suspected to be involved in water absorption in the normal rat large intestine (Calamita *et al.,* 2001). We observe that *AQP8* is significantly expressed to a higher level in the proximal human large intestine compared to the distal tissues (p<0.006, data not shown.) The family of claudin tight junction proteins may also play a role in maintaining the water barrier integrity in the large intestine (Jeansonne *et al.,* 2003). We found the expression of claudin-8 (*CLDN8*) is much more highly expressed in the distal colorectal tissues. Conversely, claudin-15 (*CLDN15*), which is also believed to be localized in the tight junction fibrils was expressed at a higher level in the proximal colorectal tissues (Colegio *et al.,* 2002).

### The nature of gene expression change along the large intestine

While one goal of this work was to understand which gene transcripts are differentially expressed along the large intestine, a second aim was to explore the nature of these expression changes along the proximal-distal axis in region or segment-specific detail.

We observed two broad patterns of statistically significant transcript expression change along the colorectum. The major pattern is described by those 65 gene transcripts that were well fitted by a two-segment expression model. We suggest that the expression of these transcripts is dichotomous in nature - elevated in the proximal segments and decreased in distal segments, or vice-versa.

Such data are consistent with the conventional anatomical view that the 'natural' divide between the proximal and distal large intestine occurs between the ascending and descending colon. This finding is contrary to a recent report by Komuro *et al.* that a breakpoint between the descending and sigmoid colon yields the largest differential expression(Komuro *et al.,* 2005). However, we note that in addition to analysing this pattern in colorectal cancer specimens, Komuro *et al.* also chose to include the transverse colon in their analysis. We intentionally exclude tissues from that segment to avoid the possible confounding affect related to the predicted midgut-hindgut fusion point approximately two-thirds the length of the transverse colon.

A second set of 50 transcripts do not display a dichotomous change, but rather show a significant improvement in fit by applying the expression data to a five-segment model supporting a more gradual expression gradient moving along the large intestine from the cecum to the rectum.

These two characteristic expression patterns hint that gene expression along the proximal-distal axis is perhaps coordinated by two underlying systems of organization.

We observed that the majority of differentially expressed transcripts in the adult normal tissues measured here are expressed in a pattern that is consistent with a midgut vs. hindgut pattern of embryonic development. Further, multivariate methods including supervised PCA and canonical variate analysis also suggest that the primary source of variation among these data are explained by the proximal vs. distal divide. In a recent study Glebov *et al.* found that the number of genes differentially expressed between the ascending and descending colon in the adult is substantially larger than the number of genes likewise identified in 17-24 week old fetal large intestines. Glebov *et al.* hypothesize that the gene expression pattern of the adult large intestine is possibly set concurrently with expression of the adult colonic phenotype at ∼30 weeks gestation or perhaps even in response to post-natal luminal contents of the gastrointestinal tract. While we did not explore gene expression in the fetal large intestine, we observe patterns of expression in the adult that support an embryonic origin consistent with the midgut-hindgut fusion.

Most of those transcripts that exhibit a gradual expression change between the cecum and rectum exhibit a prototypical pattern of increased expression moving from the cecum to the rectum. This pattern is not observed in the midgut-hindgut differential transcripts where the number transcripts elevated proximally is approximately equal to the number elevated in the distal region. We propose that the characteristic distally increasing pattern in those transcripts could be a function of extrinsic factors in comparison to the intrinsically defined midgut-hindgut pattern. Such factors could include the effect of luminal contents that move in a unidirectional manner from the cecum to the rectum and/or the regional changes in microflora along the large intestine. Further work will be required to investigate whether such extrinsic controls are working in a positive manner of inducing transcriptional activity or through a reduced transcriptional silencing.

### Gene expression changes in concert along the large intestine

To explore the expression of genes in concert along the large intestine, we also apply principal component analysis and profile analysis to these expression data. There is strong evidence for a proximal versus distal gene expression pattern with these multivariate visualization techniques. Furthermore, profile analysis, which simultaneously maximizes inter-segment expression differences while attempting to shrink the intra-segment variance, suggests that the same set of genes that account for the variability between the cecum to the rectum also best separate the individual segments. Though these multivariate results do not exclude a subtle proximal-distal gradient, the apparent bimodal nature of these multivariate plots suggests that the major source of expression variation in these tissues is consistent with a midgut- vs. hindgut-derived pattern.

### A smaller set of genes can be informative

Finally, the sophisticated classification method of support vector machines is used to select a subset of informative probesets that can be used to provide a stable, robust classification of proximal versus distal tissues. Probesets 'selected' by the SVM 1002 are a subset of the differential transcripts identified by univariate methods, above. By evaluating this 13-transcript model in the independent validation set, the robustness of these predictors is further demonstrated.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### Conclusions

Our work suggests that transcript abundance, and perhaps transcriptional regulation, follows two broad patterns along the proximal-distal axis of the large intestine. The dominant pattern is a dichotomous expression pattern consistent with the midgut-hindgut embryonic origins of the proximal and distal gut. Transcripts that follow this pattern are roughly equally split into those that are elevated distally and those elevated proximally. The second pattern we observe is characterised by a gradual change in transcript levels from the cecum to the rectum, nearly all of which exhibit increasing expression toward the distal tissues. We propose that tissues that exhibit the dichotomous midgut-hindgut patterns are likely to reflect the intrinsic embryonic origins of the large intestine while those that exhibit a gradual change reflect extrinsic factors such as luminal flow and microflora changes. Taken together, these patterns constitute a gene expression map of the large intestine. This is the first such map of an entire human organ.

**TABLE 1: List of genes differentially expressed higher in proximal tissues relative to distal tissues. (p<0.05)**

| | | | | **Proxima-Distal** | | | **Cecum-Rectum** | | | **Validation** | | | |
|---|---|---|---|---|---|---|---|---|---|---|---|---|---|
| **Rank** | **Probeset ID** | **Symbol** | **Description** | **Expr. Δ** | ***t*** | **P-Value** | **Expr. Δ** | ***t*** | **P-Value** | **P-Value** | ***t*** | **CI Low** | **CI High** |
| 1 | 222262_s_at | ETNK1 | ethanolamine kinase 1 | 3.3492 | -12.9258 | 5.27E-23 | 3.5741 | -9.0521 | 6.53E-09 | 1.37E-01 | 1.5891 | -0.3764 | 2.4320 |
| 2 | 225458_at | SEC6L1 | SEC6-like 1 (S. cerevisiae) | 5.4422 | -12.5937 | 5.10E-22 | 6.2917 | -9.2685 | 2.57E-09 | 1.75E-01 | 1.4370 | -0.7340 | 3.6253 |
| 3 | 225457_s_at | SEC6L1 | SEC6-like 1 (S. cerevisiae) | 4.2221 | -12.5347 | 7.62E-22 | 4.9764 | -9.7261 | 3.59E-10 | 2.19E-01 | 1.2930 | -0.8902 | 3.5413 |
| 4 | 219017_at | ETNK1 | ethanolamine kinase 1 | 4.0801 | -12.3947 | 1.98E-21 | 4.1238 | -8.1023 | 3.99E-07 | 2.63E-01 | 1.1704 | -1.0423 | 3.4942 |
| 5 | 207558_s_at | PITX2 | paired-like homeodomain transcription factor 2 | 1.6252 | -12.3516 | 2.66E-21 | 1.7549 | -8.5481 | 5.79E-08 | 5.20E-01 | 0.6582 | -0.6362 | 1.2099 |
| 6 | 224453_s_at | ETNK1 | ethanolamine kinase 1 | 2.0637 | -11.5429 | 6.45E-19 | 2.1692 | -8.0763 | 4.47E-07 | 2.07E-01 | 1.3638 | -0.1907 | 0.7586 |
| 7 | 229230_at | OSTalpha | organic solute transporter alpha | 2.4793 | -10.8011 | 9.47E-17 | 2.7768 | -8.6246 | 4.15E-08 | 1.95E-01 | 1.3510 | -0.4902 | 2.2212 |
| 8 | 206340_at | NR1H4 | nuclear receptor subfamily 1, group H, member 4 | 2.0505 | -10.3266 | 2.22E-15 | 2.4066 | -9.1541 | 4.20E-09 | 3.55E-02 | 2.3580 | 0.0394 | 0.9527 |
| 9 | 226432_at | | ** no description** | 2.3181 | -10.0408 | 1.46E-14 | 2.5744 | -7.2261 | 1.76E-05 | 2.49E-01 | 1.2193 | -0.5313 | 1.8442 |
| 10 | 209869_at | ADRA2A | adrenergic, alpha-2A-, receptor 1.6585 | -9.8367 | 5.55E-14 | 1.7705 | - 8.0507 | 4.99E-07 | 2.45E-01 | 1.2272 | -0.4738 | 1.6677 | |
| 11 | 227194_at | FAM3B | family with sequence similarity 3, member B | 2.8282 | -9.8079 | 6.70E-14 | 3.4326 | -6.9816 | 5.00E-05 | 2.04E-01 | 1.3699 | -0.6662 | 2.7145 |
| 12 | 207251_at | MEP1B | meprin A, beta | 1.7581 | -9.7239 | 1.16E-13 | 1.8022 | -6.5673 | 2.91E-04 | 1.52E-01 | 1.5371 | -0.2025 | 1.1482 |
| 13 | 219954_s_at | GBA3 | glucosidase, beta, acid 3 (cytosolic) | 1.7033 | -9.6737 | 1.60E-13 | 1.9800 | -8.3619 | 1.30E-07 | 1.76E-01 | 1.4742 | -0.2567 | 1.1929 |
| 14 | 219955_at | FLJ10884 | hypothetical protein FLJ10884 | 1.8400 | -9.1831 | 3.77E-12 | 1.9031 | -5.9016 | 4.66E-03 | 2.78E-01 | 1.1257 | -0.0917 | 0.2976 |
| 15 | 225290_at | | ** no description | 2.2680 | -9.1191 | 5.68E-12 | 2.4516 | -6.2630 | 1.04E-03 | 3.30E-01 | 1.0125 | -0.8929 | 2.4715 |
| 16 | 201920_at | SLC20A1 | solute carrier family 20 (phosphate transporter), member 1 | 2.1030 | -8.5555 | 1.97E-10 | 2.3428 | -7.0466 | 3.79E-05 | 3.68E-01 | 0.9338 | -1.0459 | 2.6359 |
| 17 | 206294_at | HSD3B2 | hydroxy-delta-5-steroid dehydrogenase, 3 beta- and steroid delta-isomerase 2 | 1.8455 | -8.2334 | 1.43E-09 | 2.0613 | -6.6283 | 7.75L-04 | 3.68E-01 | 0.9331 | -0.9742 | 2.4564 |
| 18 | 231576_at | | **no description** | 2.1646 | -8.0045 | 5.75E-09 | | | | 1.89E-01 | 1.4363 | -0.3026 | 1.3050 |
| 19 | 222943_at | GBA3 | glucosidase, beta, acid 3 (cytosolic) | 2.0596 | -7.9083 | 1.03E-08 | 2.5806 | -6.9404 | 5.96E-05 | 3.62E-01 | 0.9560 | -0.7354 | 1.8413 |
| 20 | 202236_s_at | SLC16A1 | solute carrier family 16 (monocarboxylic acid | 1.6747 | -7.6989 | 3.58E-08 | 1.8552 | -6.9860 | 4.91E-05 | 7.30E-01 | -0.3520 | -1.4137 | 1.0142 |
| 21 | 205366_s_at | HOXB6 | transporters), member 1 homeo box B6 | 1.4861 | -7,6727 | 4.18E-08 | 1.6332 | -6.0387 | 2.65E-03 | 3.75E-01 | 0.9368 | -0.3720 | 0.8890 |
| 22 | 222774_s_at | NETO2 | neuropilin (NRP) and tolloid (TLL)-like 2 | 1.6919 | -7.5826 | 7.11E-08 | | | | 6.56E-01 | 0.4551 | -0.5353 | 0.8246 |
| 23 | 235733_at | | ** no description | 1.1776 | -7.4926 | 1.21E-07 | 1.2384 | -6.0872 | 2.17E-03 | 7.99E-02 | 1.8733 | -0.0196 | 0.3111 |
| 24 | 202235_at | AFARP1 | family pseudogene | 1.2859 | -7.3793 | 2.33E-07 | 1.3698 | -6.6895 | 1.73E-04 | 5.44E-01 | -0.6204 | -0.9183 | 0.5044 |
| 25 | 224476_s_at | MESP1 | mesoderm posterior 1 | 1.2840 | -7.2589 | 4.68E-07 | | | | 2.16E-01 | 1.2876 | -0.0855 | 0.3497 |
| 26 | 206858_s_at | HOXC6 | homeo box C6 | 1.2640 | -7.1875 | 7.05E-07 | 1.3672 | -6.2775 | 9.82E-04 | 1.49E-01 | 1.5380 | -0.1110 | 0.6535 |
| 27 | 208126_s_at | CYP2C18 | cytochrome P450, family subfamily C, polypeptide 18 | 1.5721 | -7.0842 | 1.27E-06 | | | | 7.70E-01 | 0.2970 | -0.8071 | 1.0692 |
| 28 | 207529_at | DEFA5 | defensin, alpha 5, Paneth cell-specific | 2.8342 | -7.0313 | 1.71E-06 | 3.8363 | -5.9701 | 3.51E-03 | 1.76E-01 | 1.5002 | -0.4189 | 1.8957 |
| 29 | 209692_at | EYA2 | eyes absent homolog 2 | 1.3808 | -6.9744 | 2.36E-06 | 1.4435 | -5.9334 | 4.09E-03 | 2.40E-02 | 2.5104 | 0.0383 | 0.4702 |
| 30 | 214595_at | KCNG1 | potassium voltage-gated channel, subfamily G, member 1 | 1.1633 | -6.9706 | 2.41E-06 | 1.2868 | -6.4306 | 5.17E-04 | 9.41E-02 | -1.7744 | -0.5220 | 0.0453 |
| 31 | 202888_s_at | ANPEP | alanyl (membrane) aminopeptidase (aminopeptidase N, aminopeptidase M, microsomal aminopeptidase, CD13, p150) | 2.6011 | -6.8676 | 4.30E-06 | 3.3179 | -5.7250 | 9.58E-03 | 2.63E-01 | 1.1662 | -0.9121 | 3.0790 |
| 32 | 202718_at | IGFBP2 | insulin-like growth factor protein 2, 36kDa | 1.8892 | -6.8559 | 4.59E-06 | | | | 7.97E-01 | 0.2631 | -1.0565 | 1.3500 |
| 33 | 221804_s_at | FAM45A | family with sequence similarity 45, member A | 1.3071 | -6.8456 | 4.86E-06 | | | | 6.85E-01 | -0.4156 | -1.7005 | 1.1551 |
| 34 | 207158_at | APOBEC1 | apolipoprotein B mRNA editing enzyme, catalytic polypeptide 1 | 1.4298 | -6.7384 | 8.81E-06 | | | | 8.55E-01 | 0.1857 | -0.5250 | 0.6260 |
| 35 | 230949_at | SLC23A3 | solute carrier family 23 transporters), member 3 | 1.1622 | -6.5961 | 1.92E-05 | | | | 6.05E-02 | 2.0879 | -0.0267 | 1.0424 |
| 36 | 205541_s_at | GSPT2 | G1 to S phase transition 2 | 1.3378 | -6.5339 | 2.70E-05 | 1.4485 | -5.7155 | 9.96E-03 | 1.91E-01 | 1.4047 | -0.2567 | 1.1282 |
| 37 | 207212_at | SLC9A3 | solute carrier family 9 (sodium/hydrogen exchanger), isoform 3 | 1.2571 | -6.5310 | 2.74E-05 | | | | 9.52E-01 | 0.0608 | -0.2994 | 0.3171 |
| 38 | 215103_at | CYP2C18 | cytochrome P450, family 2, subfamily C, polypeptide 18 | 1.3638 | -6.5193 | 2.92E-05 | 1.4312 | -5.9261 | 4.21E-03 | 9.81E-01 | 0.0248 | -0.6717 | 0.6874 |
| 39 | 206755_at | CYP2B6 | cytochrome P450, family 2, subfamily B, polypeptide 6 | 1.2980 | -6.4787 | 3.64E-05 | 1.3244 | -5.5367 | 2.05E-02 | 7.86E-03 | 3,3120 | 0.1017 | 0.5198 |
| 40 | 239656_at | | **no description** | 1.1506 | -6.4761 | 3.69E-05 | | | | 5.91E-01 | 0.5545 | -0.3367 | 0.5611 |
| 41 | 222955_s_at | FAM45A | family with sequence similarity 45, member A | 1.2688 | -6.4573 | 4.09E-05 | | | | 8.98E-01 | 0.1300 | -0.2480 | 0.2802 |
| 42 | 213181_s_at | MOCS1 | molybdenum cofactor synthesis 1 | 1.1617 | -6.4528 | 4.19E-05 | 1.2410 | -6.4040 | 5.78E-04 | 8.98E-01 | 0.1300 | -0.2891 | 0.3268 |
| 43 | 205522_at | HOXD4 | homeo box D4 | 1.2966 | -6.4496 | 4.26E-05 | 1.4206 | -5.6334 | 1.39E-02 | 1.70E-02 | 2.7802 | 0.0674 | 0.5621 |
| 44 | 221304_at | UGT1A8 | UDP glycosyltransferase 1 family, polypeptide A8 | 1.3599 | -6.4054 | 5.40E-05 | | | | 3.32E-02 | 2.4124 | 0.0157 | 0.3156 |
| 45 | 205660_at | OASL | 2'-5'-oligoadenylate synthetase-like | 1.5483 | -6,3676 | 6.61E-05 | | | | 9.13E-02 | 1.8836 | -0.1619 | 1.8170 |
| 46 | 218888_s_at | | **no description** | 1.6234 | -6.3647 | 6.71E-05 | | | | 8.65E-01 | 0.1729 | -0.7162 | 0.8440 |
| 47 | 209900_s_at | SLC16A1 | solute carrier family 16 (monocarboxylic acid transporters), member 1 | 1.4721 | -6.3225 | 8.41E-05 | 1.6899 | -6.0457 | 2.57E-03 | 7.73E-01 | -0.2938 | -1.3553 | 1.0276 |
| 48 | 242059_at | | **no description** | 1.6676 | -6.3073 | 9.12E-05 | | | | 1.58E-01 | 1.5283 | -0.3359 | 1.7837 |
| 49 | 221305_s_at | UGT1A8 | UDP glycosyltransferase 1 family, polypeptide A8 | 1.6300 | -6.3057 | 9.20E-05 | | | | 1.16E-01 | 1.7472 | -0.0934 | 0.7101 |
| 50 | 219197_s_at | SCUBE2 | signal peptide, CUB EGF-like 2 | 1.2723 | -6.2538 | 1.21E-04 | 1.5426 | -7.2700 | 1.45E-05 | 1.51E-01 | 1.5707 | -0.0850 | 0.4708 |
| 51 | 236860_at | NPY6R | neuropeptide Y receptor (pseudogene) | 1.1988 | -6.2070 | 1.55E-04 | | | | 1.50E-01 | 1.5108 | -0.0514 | 0.3088 |
| 52 | 218739_at | ABHD5 | abhydrolase domain containing 5 | 1.2190 | -6.2061 | 1.56E-04 | | | | 8.25E-01 | 0.2256 | -0.4494 | 0.5557 |
| 53 | 210797_s_at | OASL | 2'-5'-oligoadenylate synthetase-like | 1.4082 | -6.1890 | 1.70E-04 | | | | 2.62E-01 | 1.1791 | -0.1607 | 0.5374 |
| 54 | 206754_s_at | CYP2B6 | cytochrome P450, family subfamily B, polypeptide 6 | 1.5418 | -6.1369 | 2.24E-04 | | | | 2.00E-01 | 1.3404 | -0.3312 | 1.4532 |
| 55 | 203333_at | KIFAP3 | kinesin-associated protein 3 | 1.2568 | -6.1317 | 2.30E-04 | | | | 5.92E-01 | 0.5550 | -0.6324 | 1.0488 |
| 56 | 224454_at | ETNK1 | ethanolamine kinase 1 | 1.1406 | -6.1181 | 2.47E-04 | | | | 3.33E-01 | 0.9980 | -0.1088 | 0.3037 |
| 57 | 214651_s_at | HOXA9 | homeo box A9 | 1.4981 | -6.0474 | 3.57E-04 | 1.6730 | -5.8388 | 6.02E-03 | 7.54E-01 | 0.3192 | -0.9026 | 1.2175 |
| 58 | 242683_at | na | hypothetical gene supported by AK095347 | 1.2426 | -5.9201 | 6.86E-04 | | | | 3.97E-02 | 2.3200 | 0.0201 | 0.6997 |
| 59 | 236894_at | | **no description** | 1.3679 | -5.8885 | 8.07E-04 | | | | 6.22E-01 | 0.5028 | -0.1866 | 0.3029 |
| 60 | 218136_s_at | MSCP | mitochondrial solute carrier protein | 1.2016 | -5.8872 | 8.12E-04 | | | | 3.93E-01 | 0.8820 | -0.1419 | 0.3403 |
| 61 | 210153_s_at | ME2 | malic enzyme 2, NAD(+)-mitochondrial | 1.2047 | -5.8498 | 9.82E-04 | | | | 6.28E-01 | 0.5001 | -0.4716 | 0.7442 |
| 62 | 209752_at | REG1A | regenerating islet-derived alpha (pancreatic stone protein, pancreatic thread protein) | 2.7216 | -5.8414 | 1.02E-03 | | | | 5.62E-01 | -0,5914 | -0.3380 | 0.1901 |
| 63 | 238638_at | SLC37A2 | solute carrier family 37 (glycerol-3-phosphate transporter), member 2 | 1.3919 | -5.8351 | 1.06E-03 | | | | 5.80E-01 | 0.5732 | -0.5148 | 0.8685 |
| 64 | 214421_x_at | CYP2C9 | cytochrome P450, family 2, subfamily C, polypeptide 9 | | | 6.79E-03 | 1.3877 | -5.8095 | 6.79E-03 | 8.26E-02 | 1.8529 | -0.0292 | 0.4316 |
| 65 | 205815_at | PAP | pancreatitis-associated protein | 2.0272 | -5.7979 | 1.28E-03 | 2.7965 | -5.5114 | 2.27E-02 | 1.36E-01 | 1.6661 | -0.1684 | 1.0163 |
| 66 | 225351_at | FAM45A | family with sequence similarity 45, member A | 1.2592 | -5.6944 | 2.14E-03 | | | | 8.22E-01 | -0.2296 | -0.9944 | 0.8026 |
| 67 | 243669_s_at | PRAP1 | proline-rich acidic protein 1 | 1.4986 | -5.6740 | 2.37E-03 | | | | 4.66E-01 | 0.7466 | -0.7334 | 1.5338 |
| 68 | 228564_at | LOC375295 | hypothetical gene supported by BC013438 | 1.1976 | -5.6664 | 2.47E-03 | | | | 5.38E-02 | 2.1149 | -0.0035 | 0.3785 |
| 69 | 223541_at | HAS3 | hyaluronan synthase 3 | 1.4178 | -5.6557 | 2.60E-03 | | | | 3.82E-01 | -0.8990 | -1.3977 | 0.5637 |
| 70 | 202234_s_at | AFARP1 | AKR7 family pseudogene | 1.4304 | -5.6464 | 2.72E-03 | | | | 7.49E-01 | 0.3259 | -1.0571 | 1.4355 |
| 71 | 203920_at | NR1H3 | nuclear receptor subfamily 1, group H, member 3 | | | 1.87E-02 | 1.3409 | -5.5600 | 1.87E-02 | 4.58E-01 | 0.7617 | -0.3137 | 0.6637 |
| 72 | 231897_at | ZNF483 | zinc finger protein 483 | 1.3192 | -5.5272 | 4.90E-03 | | | | 9.53E-01 | 0.0602 | -1.1123 | 1.1762 |
| 73 | 228155_at | C10orf58 | chromosome 10 open reading frame 58 | 1.4264 | -5.5143 | 5.21E-03 | | | | 8.53E-01 | 0.1888 | -1.3883 | 1.6572 |
| 74 | 206601_s_at | HOXD3 | homeo box D3 | 1.1325 | -5.5056 | 5.44E-03 | 1.2135 | -5.5679 | 1.81E-02 | 3.90E-01 | 0.8826 | -0.1434 | 0.3488 |
| 75 | 215913_s_at | GULP1 | GULP, engulfment adaptor PTB domain containing 1 | | | 2.39E-02 | 1.4578 | -5.4985 | 2.39E-02 | 2.46E-02 | 2.4689 | 0.0299 | 0.3831 |
| 76 | 208596_s_at | UGT1A3 | UDP glycosyltransferase 1 family, polypeptide A3 | 1.6580 | -5.3741 | 1.03E-02 | | | | 3.94E-01 | 0.8810 | -0.5799 | 1.3851 |
| 77 | 202495_at | TBCC | tubulin-specific chaperone c | 1.1465 | -5.3411 | 1.20E-02 | | | | 8.85E-01 | 0.1471 | -0.3784 | 0.4337 |
| 78 | 221920_s_at | MSCP | mitochondrial solute carrier protein | 1.1893 | -5.3370 | 1.23E-02 | | | | 3.19E-01 | 1.0688 | -0.2442 | 0.6546 |
| 79 | 223058_at | C10orf45 | chromosome 10 open reading frame 45 | 1.3829 | -5.3188 | 1.34E-02 | | | | 9.93E-01 | 0.0092 | -1.2206 | 1.2307 |
| 80 | 219926_at | POPDC3 | popeye domain containing 3 | 1.1296 | -5.2863 | 1.56E-02 | | | | 1.73E-01 | 1.4622 | -0.0737 | 0.3604 |
| 81 | 210154_at | ME2 | malic enzyme 2, NAD(+)-dependent, mitochondrial | 1.3016 | -5.2581 | 1.78E-02 | | | | 4.06E-01 | 0.8804 | -0.4040 | 0.8951 |
| 82 | 220753_s_at | CRYL1 | crystallin, lambda 1 | 1.2752 | -5.2392 | 1.95E-02 | | | | 9.42E-01 | 0.0735 | -0.9931 | 1.0643 |
| 83 | 205505_at | GCNT1 | glucosaminyl (N-acetyl) transferase 1, core 2 (beta-1,6-N-acetylglucasaminyltransfer ase) | 1.1227 | -5.2361 | 1.98E-02 | | | | 1.91E-01 | 1.3736 | -0.0833 | 0.3805 |
| 84 | 219640_at | CLDN15 | claudin 15 | 1.1692 | -5.2276 | 2.06E-02 | | | | 3.03E-01 | 1.0642 | -0.1625 | 0.4894 |
| 85 | 214038_at | CCL8 | chemokine (C-C motif) ligand 8 | 1.6140 | -5.2067 | 2.27E-02 | | | | 1.29E-01 | 1.7169 | -0.2431 | 1.5559 |
| 86 | 220017_x_at | CYP2C9 | cytochrome P450, family 2, subfamily C, polypeptide 9 | 1.3983 | -5.1902 | 2.46E-02 | 1.5251 | -5.4185 | 3.29E-02 | 1.56E-03 | 3.8998 | 0.1592 | 0.5472 |
| 87 | 206407_s_at | CCL13 | chemokine (C-C motif) ligand 13 | 1.4448 | -5.1730 | 2.66E-02 | | | | 9.06E-02 | 1.8234 | -0.0265 | 0.3189 |
| 88 | 220585_at | FLJ22761 | hypothetical protein FLJ22761 | 1.1558 | -5.1501 | 2.96E-02 | | | | 7.05E-01 | 0.3868 | -0.1662 | 0.2388 |
| 89 | 217085_at | SLC14A2 | solute carrier family 14 (urea transporter), member 2 | 1.2940 | -5.1161 | 3.47E-02 | | | | 1.69E-01 | 1.5324 | -0.3248 | 1.5282 |
| 90 | 205208_at | FTHFD | formyltetrahydrofolate dehydrogenase | 1.2531 | -5.1123 | 3.53E-02 | | | | 7.99E-01 | 0.2585 | -0.3126 | 0.3997 |
| 91 | 203639_s_at | FGFR2 | fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome) | 1.2760 | -5.0917 | 3.89E-02 | | | | 3.02E-01 | 1.0705 | -0.1918 | 0.5747 |
| 92 | 204663_at | ME3 | malic enzyme 3, NADP(+)-dependent, mitochondrial | 1.1447 | -5.0447 | 4.83E-02 | | | | 5.46E-01 | 0.6203 | -0.3844 | 0.6922 |
| 93 | 211776_s_at | EPB41L3 | erythrocyte membrane band 4,1-like 3 | 1.2553 | -5.0391 | 4.95E-02 | | | | 5.81E-01 | 0.5706 | -0.4283 | 0.7236 |

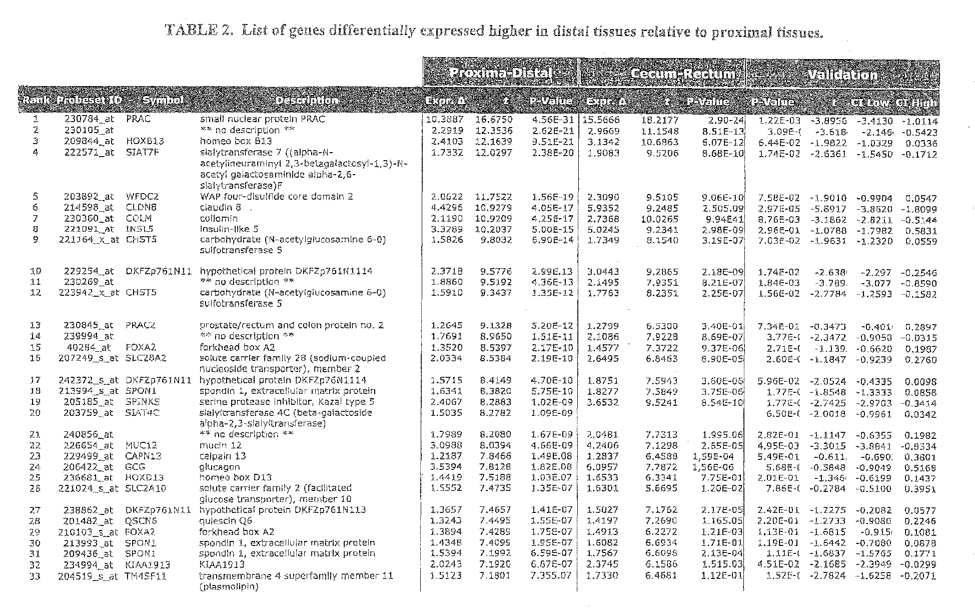

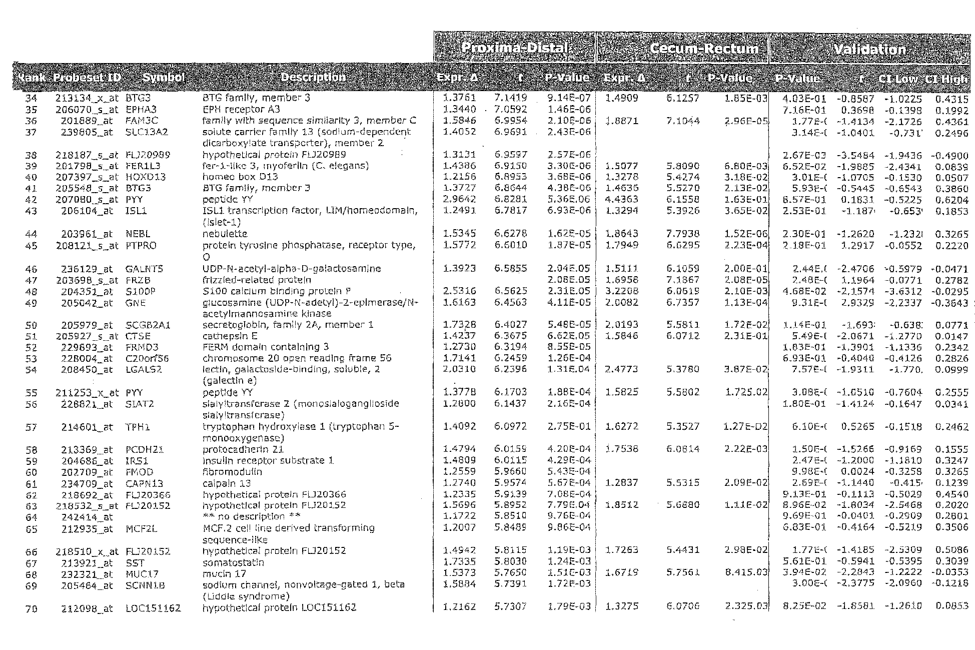

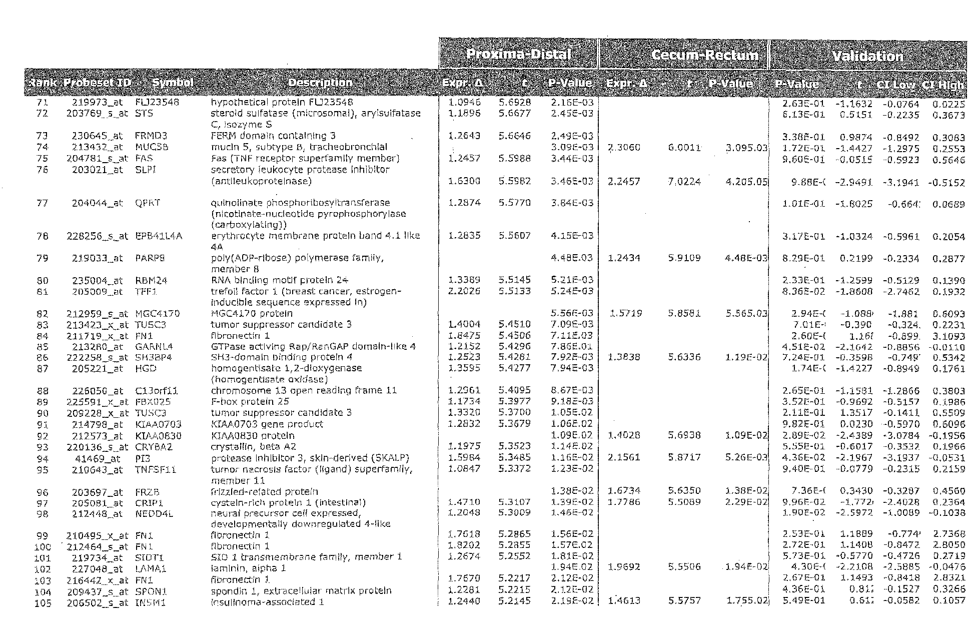

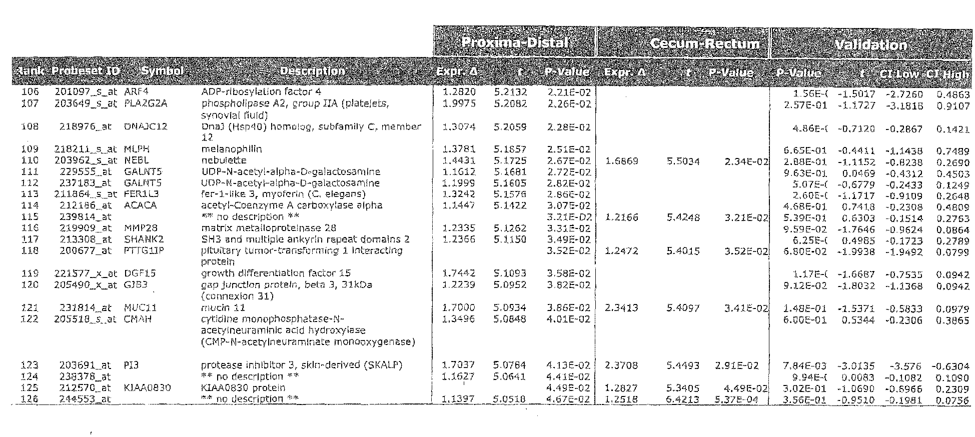

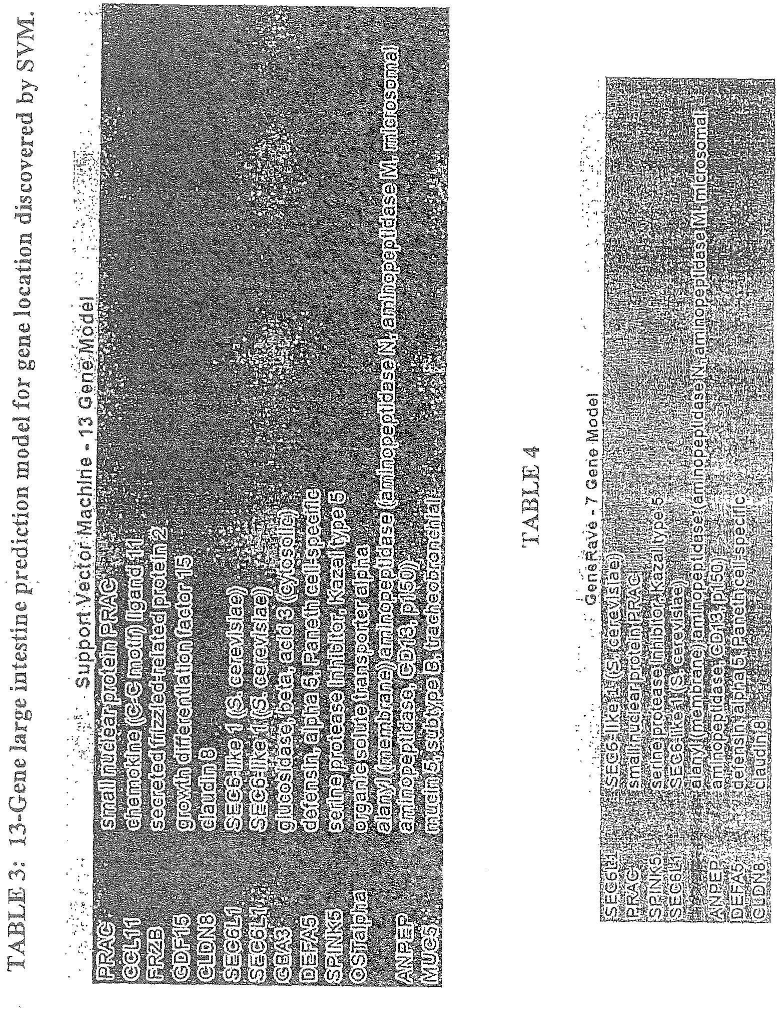

**TABLE 5: GeneRave models for Prox vs Distal**

| | **X-Validated error** | **Model** | **SENS** | **SPEC** | **PPV** | **NPV** | **LRP** | **LRN** |
|---|---|---|---|---|---|---|---|---|
| 200832_s_at,SCD 204580_at,MMP12 206637_at,P2RY14 214598_at,CLDN8 219017_at,ETNK1 | 10.828 | 1 | 0.989 | 0.968 | 0.979 | 0.984 | 30.674 | 0.011 |
| 205549_at,PCP4 207249_s_at,SLC28A2 209924_at,CCL18 219140_s_at,RBP4 225458_at,DKFZP564I1171_230784_at,PRAC | 12.10191 | 2 | 0.947 | 0.952 | 0.968 | 0.922 | 19.579 | 0.055 |
| 201123_s_at,EIF5A 202718_atIGFBP2 221577_x_at,GDF15 225457_s_at,DKFZP564I1171 226654_at,MUC12 | 10.82803 | 3 | 0.989 | 0.919 | 0.949 | 0.983 | 12.269 | 0.011 |
| 209728_at,HLA-DRB4 209844_at,HOXB13 221091_at,INSL5 222262_s_at,ETNK1 | 114.46497 | 4 | 0.926 | 0.919 | 0.946 | 0.891 | 11.486 | 0.080 |
| 202888_s_at,ANPEP 207529_at,DEFA5 221164_x_at,CHST5 226432_at,NA 230360_at,COLM | 14.64968 | 5 | 0.958 | 0.935 | 0.958 | 0.935 | 14.847 | 0.045 |
| 205464_at,SCNN41B 21 1719_x_at,FN1 224453_s_at,ETNK1 225290_at,NA 229230_at,OSTalpha 229400_at,HOXD10 230269_at,NA | 15.92357 | 6 | 0.958 | 0.903 | 0.938 | 0.933 | 9.898 | 0.047 |
| 201920_at,SLC20A1 211969_at,HSPCA 217320_at,NA 32128_at,CCL18 230105_at,HOXB13 | 17.83439 | 7 | 0.947 | 0.919 | 0.947 | 0.919 | 11.747 | 0.057 |
| 209795_at,CD69 212768_s_at,OLFM4 215125_s_at,UGT1A6 223942_x_at,CHST5 231576_at,NA 231814 _at,MUC11 | 18.47134 | 8 | 0.937 | 0.919 | 0.947 | 0.905 | 11.617 | 0.069 |
| 203649_s_at,PLA2G2A 205815_at,REG3A 206407_s_at,CCL13 206422_at,GCG 208596_s_at,UGT1A3 210495_x_at,FN1 217546_at,MT1M236121_at,OR51E2 | 18.47134 | 9 | 0.947 | 0.935 | 0.957 | 0.921 | 14.684 | 0.056 |
| 202236_s_at,SLC16A1 203892_at,WFDC2 204351_at,S100P 208121_s_at,PTPRO 210133_at,CCL11 219087_atASPN 227194_at,FAM3B | 14.01174 | 10 | 0.947 | 0.968 | 0.978 | 0.923 | 29.368 | 0.054 |
| 201324_at,EMP1 203962_s_at,NEBL 205009_at,TFF1 205518_s_at,CMAH 207080_s_at,PYY 219955_at,ECAT11 222774_s_at,NETO2 | 0.178439 | 11 | 0.947 | 0.919 | 0.947 | 0.919 | 11.747 | 0.057 |
| 204818_at,HSD17B2 20522_1_at,HGD 205950_s_at,CA1 206100_at,CPM 208450_at,LGALS2 214973_x_at,IGHD 216442_x_ at,FN1 | 15.28662 | 12 | 0.958 | 0.919 | 0.948 | 0.934 | 11.878 | 0.046 |
| 206207_at,CLC 207814_at,DEFA6 212464_s_at,FN1 226847_at,FST 236513_at,NA 240856_at,NA 242059_at,ETNK1 | 18.47134 | 13 | 0.937 | 0.919 | 0.947 | 0.905 | 11.617 | 0.069 |
| 207558_s_at,PITX2 224009_x_at,DHRS9 229254 at,DKFZp761N1114 234994_at,KIAAI913 | 17.83439 | 14 | 0.947 | 0.871 | 0.918 | 0.915 | 7.342 | 0.060 |
| 205498_at,GHR 206294_at,HSD3B2 207251_at,MEP1B 214651_s_at,HOXA9 224412_s_at,TRPM6 239994_at,NA | 18.47134 | 15 | 0.968 | 0.903 | 0.939 | 0.949 | 10.007 | 0.035 |
| 205185_at,SPINK5 208383_s_at,PCK1 209869_at,ADRA2A 10519_s_at,NQO1 222943_at,GBA3 228004_at,NA | 20.38217 | 16 | 0.947 | 0.887 | 0.928 | 0.917 | 8.391 | 0.059 |
| 205979_at,SCGB2A1 206340_at,NR1H4 218888_s_at,NETO2 22571_at,ST6GALNAC6 | 17.19745 | 17 | 0.926 | 0.887 | 0.926 | 0.887 | 8.205 | 0.083 |
| 203961_at,NEBL 204304_s_at,PROM1 209173_at,AGR2 209752_at,REG1A 221305_s_at,UGT1A8 242372_s_at,DKFZp761N11H4 | 31.21019 | 18 | 0.926 | 0.919 | 0.946 | 0.891 | 11.486 | 0.080 |
| 201963_at,ACSL1 203759_at,ST3GAL4 219954_s_at,GBA3 221024_s_at,SLC2A10 223952_x_at,DHRS9 227048 _at,LAMA1 | 14.64968 | 19 | 0.958 | 0.887 | 0.929 | 0.932 | 8.484 | 0.047 |
| 202023_at,EFNA1 202946_s_at,BTBD3 203691_at,PI3 209994_s_at,ABCB1 223058_at,C10orf45 228241_at,BCMP11 229070_at,C6orf105 234709_at,CAPN13 235706_at,CPM 236141 at,NA 238143 at,NA | 17.83439 | 20 | 0.979 | 0.984 | 0.989 | 0.968 | 60.695 | 0.021 |

### BIBLIOGRAPHY

Affymetrix. 2001a. GeneChip Expression Analysis Data Analysis Fundamentals.
Affymetrix. 2001b. Statistical Algorithms Reference Guide.
Affymetrix. 2004. Gene Expression Analysis: Technical Manual. 701021 Rev 5.
Alon, A., Barkai, N., Notterman, D.A., Gish, K., Ybarra, S., Mach, D. and Levine, A.J. Broad patterns of gene expression revealed by clustering analysis of tumor and normal colon tissues probed by oligonucleotide arrays. Proc. Natl. Acad. Sci. USA: 96, 6745-6750, June 1999
Ausubel, F. et al., "Current Protocols in Molecular Biology", John Wiley & Sons, 1998
Bair, E., T. Hastie, P. Debashis and R. Tibshirani. 2004. Prediction by supervised principal components. Stanford University
Bara, J., J. Nardelli, C. Gadenne, M. Prade and P. Burtin. 1984. Differences in the expression of mucus-associated antigens between proximal and distal human colon adenocarcinomas. Br J Cancer 49:495-501.
Bates, M.D., C.R. Erwin, L.P. Sanford, D. Wiginton, J.A. Bezerra, L.C. Schatzman, A.G. Jegga, C. Ley-Ebert, S.S. Williams, K.A. Steinbrecher, B.W. Warner, M.B. Cohen and B.J. Aronow. 2002. Novel genes and functional relationships in the adult mouse gastrointestinal tract identified by microarray analysis. Gastroenterology 122:1467-1482.
Birkenkamp-Demtroder, K., S.H. Olesen, F.B. Sorensen, S. Laurberg, P. Laiho, L.A. Aaltonen and T.F. Orntoft. 2005. Differential gene expression in colon cancer of the caecum versus the sigmoid and rectosigmoid. Gut 54:374-384.
Bonithon-Kopp, C. and A.M. Benhamiche. 1999. Are there several colorectal cancers? Epidemiological data. Eur J Cancer Prev 8 Suppl 1:S3-12.
Bonner T.I., Brenner D.J., Neufeld B.R. and Britten R.J. (1973) Reduction in the rate of DNA reassociation by sequence divergence. J Mol. Biol. 81:123-125
Bufill, J.A. 1990. Colorectal cancer: evidence for distinct genetic categories based on proximal or distal tumor location. Ann Intern Med 113:779-788.
Byrd, J.C. and R.S. Bresalier. 2004. Mucins and mucin binding proteins in colorectal cancer. Cancer Metastasis Rev 23:77-99.
Calamita, G., A. Mazzone, A. Bizzoca, A. Cavalier, G. Cassano, D. Thomas and M. Svelto. 2001. Expression and immunolocalization of the aquaporin-8 water channel in rat gastrointestinal tract. Eur J Cell Biol 80:711-719.
Caldero, J., E. Campo, C. Ascaso, J. Ramos, M.J. Panades and J.M. Rene. 1989. Regional distribution of glycoconjugates in normal, transitional and neoplastic human colonic mucosa. A histochemical study using lectins. Virchows Arch A Pathol Anat Histopathol 415:347-356.
Chalmers, A.D., J.M. Slack and C.W. Beck. 2000. Regional gene expression in the epithelia of the Xenopus tadpole gut. Mech Dev 96:125-128.
Chen, M., Y. Yang, E. Braunstein, K.E. Georgeson and C.M. Hannon. 2001. Gut expression and regulation of FAT/CD36: possible role in fatty acid transport in rat enterocytes. Am J Physiol Endocrinol Metab 281:E916-23.
Colegio, O.R., C.M. Van Itallie, H.J. McCrea, C. Rahner and J.M. Anderson. 2002. Claudius create charge-selective channels in the paracellular pathway between epithelial cells. Am J Physiol Cell Physiol 283:C142-7.
Cristianini, N. and J. Shawe-Taylor. 2000. An Introduction to Support Vector Machines and Other Kernel-based Learning Methods.
Cristianini, N., Shawe-Taylor, J. Support Vector Machines, 2000, Cambridge University Press. Cambridge.
Cuff, M.A., D.W. Lambert and S.P. Shirazi-Beechey. 2002. Substrate-induced regulation of the human colonic monocarboxylate transporter, MCT1. J Physiol 539:361-371.
de Santa Barbara, P., G.R. van den Brink and D.J. Roberts. 2003. Development and differentiation of the intestinal epithelium. Cell Mol Life Sci 60:1322-1332.
Deng, G., E. Peng, J. Gum, J. Terdiman, M. Sleisenger and Y.S. Kim. 2002. Methylation of hMLH1 promoter correlates with the gene silencing with a region-specific manner in colorectal cancer. Br J Cancer 86:574-579.
DeRisi, et al., Nature Genetics, 14:457-460 (1996
Distler, P. and P.R. Holt. 1997. Are right- and left-sided colon neoplasms distinct tumors? Dig Dis 15:302-311.
Drmanac R., Labat I. and Crkvenjakov R., An algorithm for the DNA sequence generation from k-tuple word contents of the minimal number of random fragments. J. Biomol. Struc. & Dyn. 5:1085-1102, 1991
Filipe, M.I. and A.C. Branfoot. 1976. Mucin histochemistry of the colon. Curr Top Pathol 63:143-178.
Fleming, R.E., S. Parkkila, A.K. Parkkila, H. Rajaniemi, A. Waheed and W.S. Sly. 1995. Carbonic anhydrase IV expression in rat and human gastrointestinal tract regional, cellular, and subcellular localization. J Clin Invest 96:2907-2913.
Garcia-Hirschfeld Garcia, J., A. Blanes Berenguel, L. Vicioso Recio, A. Marquez Moreno, J. Rubio Garrido and A. Matilla Vicente. 1999. Colon cancer: p53 expression and DNA ploidy. Their relation to proximal or distal tumor site. Rev Esp Enferm Dig 91:481-488.
Gautier, L., L. Cope, B.M. Bolstad and R.A. Irizarry. 2004. affy--analysis of Affymetrix GeneChip data at the probe level. Bioinformatics 20:307-315.
Gentleman, R.C., V.J. Carey, D.M. Bates, B. Bolstad, M. Dettling, S. Dudoit, B. Ellis, L. Gautier, Y. Ge, J. Gentry, K. Hornik, T. Hothorn, W. Huber, S. Iacus, R. Irizarry, F. Leisch, C. Li, M. Maechler, A.J. Rossini, G. Sawitzki, C. Smith, G. Smyth, L. Tierney, J.Y. Yang and J. Zhang. 2004. Bioconductor: open software development for computational biology and bioinformatics. Genome Biol 5:R80.
Germer S, Holland MJ, Higuchi R. 2000, High-throughput SNP allele-frequency determination in pooled DNA samples by kinetic PCR. Genome Res. 10(2):258-66.
Glebov, O.K., L.M. Rodriguez, K. Nakahara, J. Jenkins, J. Cliatt, C.J. Humbyrd, J. DeNobile, P. Soballe, R. Simon, G. Wright, P. Lynch, S. Patterson, H. Lynch, S. Gallinger, A. Buchbinder, G. Gordon, E. Hawk and I.R. Kirsch. 2003. Distinguishing right from left colon by the pattern of gene expression. Cancer Epidemiol Biomarkers Prev 12:755-762.
Gum, J.R.J., S.C. Crawley, J.W. Hicks, D.E. Szymkowski and Y.S. Kim. 2002. MUC17, a novel membrane-tethered mucin. Biochem Biophys Res Commun 291:466-475.
Guo Z, Guilfoyle RA, Thiel AJ, Wang R, Smith LM. 1994, Direct fluorescence analysis of genetic polymorphisms by hybridization with oligonucleotide arrays on glass supports. Nucleic Acids Res. 22(24):5456-65
Hastie, T, Tibshirani, R, Friedman, J, The Elements of Statistical Learning, Springer, 2001. New York. 'Chapter 4: Linear Methods for Classification'.Hostikka, S.L. and M.R. Capecchi. 1998. The mouse Hoxc11 gene: genomic structure and expression pattern. Mech Dev 70:133-145.
Hubbell, E., W.M. Liu and R. Mei. 2002. Robust estimators for expression analysis. Bioinformatics 18:1585-1592.
Iacopetta, B. 2002. Are there two sides to colorectal cancer? Int J Cancer 101:403-408.
Irizarry, R.A., B.M. Bolstad, F. Collin, L.M. Cope, B. Hobbs and T.P. Speed. 2003. Summaries of Affymetrix GeneChip probe level data. Nucleic Acids Res 31:e15.
James, R., T. Erler and J. Kazenwadel. 1994. Structure of the murine homeobox gene cdx-2. Expression in embryonic and adult intestinal epithelium, J Biol Chem 269:15229-15237.
Jeansonne, B., Q. Lu, D.A. Goodenough and Y.H. Chen. 2003. Claudin-8 interacts with multi-PDZ domain protein 1 (MUPP1) and reduces paracellular conductance in epithelial cells. Cell Mol Biol (Noisy-le-grand) 49:13-21.
Kiiveri, H. T. A bayesian approach to variable selection when the number of variables is very large Science and Statistics: A Festschrift for Terry Speed, 2003 Institute of Mathematical Statistics, Lecture Notes-Monograph Series, Vol. 3, pages 127-143
Kiiveri, H., Thomas, M., Dunne, R., *Method and Apparatus for Identifying Diagnostic Components of Asystem with a characteristic response,* International Patent Application No. PCT/AU2002/000934
Komuro, K., M. Tada, E. Tamoto, A. Kawakami, A. Matsunaga, K. Teramoto, G. Shindoh, M. Takada, K. Murakawa, M. Kanai, N. Kobayashi, Y. Fujiwara, N. Nishimura, J. Hamada, A. Ishizu, H. Ikeda, S. Kondo, H. Katoh, T. Moriuchi and T. Yoshiki. 2005. Right- and left-sided colorectal cancers display distinct expression profiles and the anatomical stratification allows a high accuracy prediction of lymph node metastasis. J Surg Res 124:216-224.
Kondo, T., P. Dolle, J. Zakany and D. Duboule. 1996. Function of posterior HoxD genes in the morphogenesis of the anal sphincter. Development 122:2651-2659.
Kosaki, K., R. Kosaki, T. Suzuki, H. Yoshihashi, T. Takahashi, K. Sasaki, M. Tomita, W. McGinnis and N. Matsuo. 2002. Complete mutation analysis panel of the 39 human HOX genes. Teratology 65:50-62.
Krzanowski, W and Marriott, F, Multivariate Analysis Part II. Classification Covariance Structures and Repeated Measures. 1995. Oxford Univ Press. Oxford. UK.Lipshutz, R.J., S.P. Fodor, T.R. Gingeras and D.J. Lockhart. 1999. High density synthetic oligonucleotide arrays. Nat Genet 21:20-24.
Liu, X.F., P. Olsson, C.D. Wolfgang, T.K. Bera, P. Duray, B. Lee and I. Pastan. 2001. PRAC: A novel small nuclear protein that is specifically expressed in human prostate and colon. Prostate 47:125-131.
Macfarlane, G.T., G.R. Gibson and J.H. Cummings. 1992. Comparison of fermentation reactions in different regions of the human colon. J Appl Bacteriol 72:57-64.
Maskos and Southern, Nuc. Acids Res. 20:1679-84, 1992
Miklos, G.L. and R. Maleszka. 2004. Microarray reality checks in the context of a complex disease. Nat Biotechnol 22:615-621.
Montgomery, R.K., A.E. Mulberg and R.J. Grand. 1999. Development of the human gastrointestinal tract: twenty years of progress. Gastroenterology 116:702-731.
Moore, A., Basilion, J., Chiocca, e., and Weissleder, R., Measuring Transferrin Receptor Gene Expression by NMR Imaging. BBA, 1402:239-249, 1988
Ortega-Cava, C.F., S. Ishihara, M.A. Rumi, K. Kawashima, N. Ishimura, H. Kazumori, J. Udagawa, Y. Kadowaki and Y. Kinoshita. 2003. Strategic compartmentalization of Toll-like receptor 4 in the mouse gut. J Immunol 170:3977-3985.
Park, Y.K., J.L. Franklin, S.H. Settle, S.E. Levy, E. Chung, L.H. Jeyakumar, Y. Shyr, M.K. Washington, R.H. Whitehead, B.J. Aronow and R.J. Coffey. 2005. Gene expression profile analysis of mouse colon embryonic development. Genesis 41:1-12.
Pease AC, Solas D, Sullivan EJ, Cronin MT, Holmes CP, Fodor SP., 1994, Light-generated oligonucleotide arrays for rapid DNA sequence analysis. Proc Natl Acad Sci U.S A. 91(11):5022-6
Peifer, M. 2002. Developmental biology: colon construction. Nature 420: 274-5, 277.
Pevzner PA., 1989, 1-Tuple DNA sequencing: computer analysis., J Biomol Struct Dyn. 7(1):63-73
Pevzner PA, Lysov YuP, Khrapko KR, Belyavsky AV, Florentiev VL, Mirzabekov AD., 1991, Improved chips for sequencing by hybridization., J Biomol Struct Dyn. 9(2):399-410
R: A Language and Environment for Statistical Computing, R Development Core Team, R Foundation for Statistical Computing, Vienna, Austria,
2007, ISBN 3-900051-07-0.
Rajendran, V.M., J. Black, T.A. Ardito, P. Sangan, S.L. Alper, C. Schweinfest, M.
Kashgarian and H.J. Binder. 2000. Regulation of DRA and AE1 in rat colon by dietary Na depletion. Am J Physiol Gastrointest Liver Physiol 279:G931-42.
Ripley, B D, Cambridge Univ Press. 1996. Pattern Recognition and Neural Networks. 'Chapter 6: Non-parametric methods.'
Sano T, Cantor CR., 1991, A streptavidin-protein A chimera that allows one-step production of a variety of specific antibody conjugates., Biotechnology (N Y), 9(12):1378-81
Schena, et al. Science 270:467-470, 1995
Scholkopf, B, Tsuda, K, and Vert, J P Kernel Methods in Computational Biology. 2004. MIT Press. Cambridge MA.
Silberg, D.G., G.P. Swain, E.R. Suh and P.G. Traber. 2000. Cdx1 and cdx2 expression during intestinal development. Gastroenterology 119:961-971.
Singh, S., R. Poulsom, A.M. Hanby, L.A. Rogers, N.A. Wright, M.C. Sheppard and M.J. Langman. 1998. Expression of oestrogen receptor and oestrogen-inducible genes pS2 and ERD5 in large bowel mucosa and cancer. J Pathol 184:153-160.
Smith SB, Finzi L, Bustamante C., 1992, Direct Mechanical Measurements of the Elasticity of Single DNA Molecules by Using Magnetic Beads, Science 258:1122-1126
Smyth, G. 2005. Limma: linear models for microarray data. In Bioinformatics and Computational Biology Solutions using R and Bioconductor. (eds. Gentleman, R., V. Carey, S. Dudoit, R. Irizarray and W. Huber), pp. 397-420. Springer, New York.
Traber, P.G. 1999. Transcriptional regulation in intestinal development. Implications for colorectal cancer. Adv Exp Med Biol 470:1-14.
Urdea et al., Nucleic Acids Symp. Ser., 24:197-200, 1991
Venables, W. and Ripley, B. D.,, Modern Applied Statistics with S, Springer-Verlag. New York, 2002.
Wedemeyer, N., Potter, T., Wetzlich, S. and Gohde, W. Flow Cytometric Quantification of Competitive Reverse Transcriptase-PCR products, Clinical Chemistry 48:9 1398-1405, 2002
Weissleder, R., Moore, A., Ph.D., Mahmood-Bhorade, U., Benveniste, H., Chiocca, E.A., Basilion, J.P. High resolution in vivo imaging of transgene expression, Nature Medicine 6:351-355, 2000
Williams, S.J., M.A. McGuckin, D.C. Gotley, H.J. Eyre, G.R. Sutherland and T.M. Antalis. 1999. Two novel mucin genes down-regulated in colorectal cancer identified by differential display. Cancer Res 59:4083-4089.
Wilson, C. and C.J. Miller. 2005. Simpleaffy: a BioConductor package for Affymetrix quality control and data analysis. Bioinformatics
Yamada, T. and D.H. Alpers. 2003. Textbook of Gastroenterology, 2 Vol. Set.

## Claims

1. A method for determining the anatomical origin of a cell or cellular population derived from either the proximal or distal region of the large intestine of an individual, said method comprising measuring the level of expression of one or more gene or genes selected from:
(i) the gene or genes detected by Affymetrix probe number 225457_s_at; and
(ii) the gene or genes detected by Affymetrix probe number 225458_at;
in said cell or cellular population wherein a higher level of expression of the genes relative to normal distal large intestine control levels is indicative of a proximal large intestine origin, and wherein said proximal region comprises the cecum and the ascending colon and said distal region comprises the splenic flexure, descending colon, sigmoid flexure and rectum.

2. The method according to claim 1 wherein said cell or cellular population is derived from a biological sample such as a feceal sample, enema wash, surgical resection, tissue biopsy, cellular material or biofluids.

3. The method according to any one of claims 1-2 wherein said level of expression is mRNA expression.

4. A method for determining the onset or predisposition to the onset of a cellular abnormality or a condition **characterized by** a cellular abnormality in the large intestine said method comprising determining, in accordance with the method of any one of claims 1 to 3, the proximal-distal gene expression profile of a biological sample derived from a known proximal or distal origin in the large intestine, wherein the detection of a gene expression profile which is inconsistent with the normal proximal-distal large intestine gene expression profile is indicative of the abnormality of the cell or cellular population expressing said profile.

## Patentansprüche

1. Verfahren zur Bestimmung des anatomischen Ursprungs einer Zelle oder einer Zellpopulation, die entweder aus dem proximalen oder dem distalen Bereich des menschlichen Dickdarms stammt, wobei das Verfahren das Messen des Expressionsspiegels eines Gens bzw. mehrerer Gene, ausgewählt aus:
(i) dem bzw. den mittels Affymetrix-Sonde Nr. 225457_s_at nachgewiesenen Gen(en); und
(ii) dem bzw. den mittels Affymetrix-Sonde Nr. 225458_at nachgewiesenen Gen(en);
in der Zelle oder der Zellpopulation umfasst, wobei ein höherer Expressionsspiegel der Gene im Vergleich zu normaler Kontrollspiegel aus dem distalen Dickdarm die Herkunft aus dem proximalen Dickdarm anzeigt; und wobei der proximale Bereich das Zäkum und das aufsteigende Kolon umfasst und der distale Bereich die Milzflexur, das absteigende Kolon, die S-Flexur und das Rektum umfasst.

2. Verfahren gemäß Anspruch 1, bei dem die Zelle oder die Zellpopulation aus einer biologischen Probe stammt, zum Beispiel einer Stuhlprobe, einer Darmspülung, einer chirurgischen Resektion, einer Gewebebiopsie, Zellmaterial oder biologischen Flüssigkeiten.

3. Verfahren gemäß einem der Ansprüche 1 bis 2, bei dem es sich bei dem Expressionsspiegel um mRNA Expression handelt.

4. Verfahren zur Bestimmung des Auftretens oder der Neigung zum Auftreten einer Zellabnormität oder einer von einer Zellabnormität im Dickdarm gekennzeichneten Erkrankung, wobei das Verfahren das Bestimmen, gemäß dem Verfahren nach einem der Ansprüche 1 bis 3, des proximal-distalen Genexpressionsprofils einer biologischen Probe umfasst, welche einer bekannten proximalen oder distalen Ursprungsstelle im Dickdarm entstammt, wobei der Nachweis eines Genexpressionsprofils, das nicht mit dem normalen proximal-distalen Genexpressionsprofil im Dickdarm übereinstimmt, einen Hinweis auf die Abnormität der Zelle oder der Zellpopulation, die dieses Profil exprimiert, darstellt.

## Revendications

1. Procédé de détection permettant la détermination de l'origine anatomique d'une cellule ou d'une population cellulaire dérivée soit de la région proximale soit de la région distale du gros intestin d'un individu, ledit procédé comprenant la mesure du niveau d'expression d'un ou de plusieurs gènes ou de gènes sélectionnés dans :
(i) le gène ou les gènes détecté (s) par la sonde Affymetrix n° 225457_s_at ; et
(ii) le gène ou les gènes détecté(s) par la sonde Affymetrix n° 225458_at ;
dans ladite cellule ou population cellulaire dans laquelle un niveau plus élevé d'expression des gènes par rapport aux niveaux de contrôle du gros intestin distal normal est représentatif d'une origine du gros intestin proximal, et dans laquelle ladite région proximale comprend le caecum et le côlon ascendant et ladite région distale comprend l'angle gauche du côlon, le côlon descendant, le côlon sigmoïde, et le rectum.

2. Procédé selon la revendication 1 dans lequel ladite cellule ou population cellulaire est dérivée d'un échantillon biologique, tel qu'un échantillon fécal, un lavement, une résection chirurgicale, une biopsie tissulaire ou des tissus, une matière cellulaire ou des biofluides.

3. Procédé selon l'une quelconque des revendications 1-2 dans lequel ledit niveau d'expression est une expression ARNm.

4. Procédé de détermination du début ou de la prédisposition à une anomalie cellulaire ou d'une condition **caractérisée par** une anomalie cellulaire dans le gros intestin, ledit procédé comprenant, la détermination, conformément au procédé de l'une quelconque des revendications 1 à 3, du profil d'expression des gènes proximal-distal d'un échantillon biologique dérivé d'une origine proximale ou distale dans le gros intestin, dans lequel la détection d'un profil d'expression de gènes, incompatible avec le profil d'expression de gènes du gros intestin proximal-distal normal, est représentative de l'anomalie de la cellule ou de la population cellulaire exprimant ledit profil.
